# EUROPEAN PATENT APPLICATION

(11) **EP 4 714 935 A1**
(43) Date of publication of application: **25.03.2026**
(21) Application number: 24807272.0
(22) Date of filing: 16.05.2024
(51) Int. Cl.: C07C 69/753, C07C 233/62, C07C 233/65, H10K 50/858, H10K 59/10, H10K 85/60

(54) **AROMATIC COMPOUND, ORGANIC ELECTROLUMINESCENT ELEMENT, AND ELECTRONIC APPARATUS**

(30) Priority: 18.05.2023 JP 2023082070
(71) Applicant: Hodogaya Chemical Co., Ltd., Tokyo, 105-0021 (JP)
(72) Inventor: HIRAI, Hiroki, Tokyo 105-0021 (JP); CHA, Hyun-Wook, Tokyo 105-0021 (JP); KIM, Hee-Jae, Tokyo 105-0021 (JP); KASE, Kouki, Tokyo 105-0021 (JP); HIRAYAMA, Yuta, Tokyo 105-0021 (JP); HAYASHI, Shuichi, Tokyo 105-0021 (JP)
(74) Representative: Müller-Boré & Partner Patentanwälte PartG mbB
(86) International application number: PCT/JP2024/018212
(87) International publication number: WO 2024/237322

(57) **Abstract**

The aromatic compound represented by the following general formula (a) or (b) has a low refractive index, and therefore, by stacking a low refractive index layer containing the aromatic compound and a high refractive index layer to use the stack as a capping layer, an organic EL device with improved light extraction efficiency can be realized. A₁ and A₂ each represent an alkanediyl, cycloalkanediyl or fluorenediyl group, L₁ to L₄ each represent a single bond, -O-, -NH-, or an alkanediyl group, Cy₁ to Cy₄ each represent an alkyl group, a cycloalkyl group, or a monovalent aromatic hydrocarbon group, and X₁ to X₄ each represent -O- or -NH-.

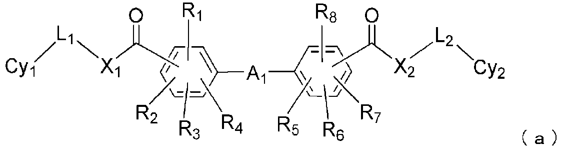

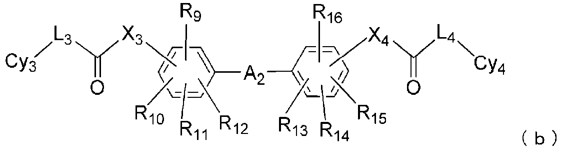

## Description

### Technical Field

The present invention relates to an aromatic compound suitable as a material for organic electroluminescent devices (hereinafter abbreviated as organic EL devices), which are self-luminous devices suitable for various display devices, and for electronic devices. In detail, the invention relates to an aromatic compound with excellent low refractive index properties, and also relates to an organic EL device and an electronic device using the aromatic compound.

### Background Art

In 1987, C. W. Tang et al. of Eastman Kodak Company made organic EL devices using organic materials practical by developing a stack structure device in which various roles are assigned to individual materials. They stack a phosphor that can transport electrons and an organic material that can transport holes, and inject both charges into the phosphor layer to emit light, thus obtaining a high luminance of 1000 cd/m² or more at a voltage of 10 V or less (see PTL 1 and PTL 2).

Recently, top-emission type light-emitting devices, in which a metal with a high work function is used as the anode and light is emitted from the top, have come into use. The bottom-emission structure, which takes out light from the bottom having a pixel circuit, limits the light-emitting area, whereas the top-emission type light-emitting device has the advantage of taking out light from the top, allowing a wider light-emitting area because the pixel circuit does not block the light from the top. In top-emission type light-emitting devices, a semi-transparent electrode such as LiF/Al/Ag (see, for example, NPL 1), Ca/Mg (see NPL 2), or LiF/MgAg is used as the cathode.

In such light-emitting devices, in the case where the light emitted by the light-emitting layer is incident on the other film, and where the light is incident at an angle greater than a certain angle, the light is totally reflected at the interface between the light-emitting layer and the other film. As a result, only a portion of the emitted light was available for use. In recent years, light-emitting devices with a "capping layer" with a high refractive index on the outside of a semi-transparent electrode with a low refractive index have been proposed to improve light extraction efficiency (see, for example, NPL 1 and PTLs 2, 4 and 5).

Also, an organic optical device, in which a low refractive index layer formed by co-evaporation of an organic semiconductor material with an additive for refractive index adjustment and a high refractive index layer are stacked , and effective light propagation control is achieved by utilizing the light interference effect of the stacked film, has been proposed (see PTL 3).

### Citation List

### Patent Literature

PTL 1: JPH8-048656A
PTL 2: JP3194657B
PTL 3: JP6210473B
PTL 4: WO2021/140896A1
PTL 5: WO2015/001726A1
NPL 1: Appl. Phys. Let., 78, 544 (2001)
NPL 2: Appl. Phys. Let., 82, 466 (2003)

### Summary of Invention

### Technical Problem

As described above, a technique has been proposed to control light propagation in organic EL devices by combining a low refractive index layer with a high refractive index layer. However, the low refractive index layer here is formed by co-evaporation of an organic semiconductor material and an additive, which complicates control of the additive amount and the deposition conditions. Therefore, there is a need to develop materials that exhibit excellent low refractive index properties even when deposited alone.

In order to solve these problems of the related-art technology, the inventors have made a diligent effort to provide a material having excellent low refractive index properties. In addition, the inventors have made a diligent study with the aim of providing an organic EL device with high light emission efficiency.

### Solution to Problem

In order to achieve the above-mentioned purpose, the inventors have made a diligent study and, as a result, have found that an aromatic compound having a structure, in which two phenylene groups having a substituent containing an amide structure or a substituent containing an ester structure bond to an alkanediyl, a cycloalkanediyl, or a fluorenediyl, exhibits excellent low refractive index properties. With that, the inventors have found that, by providing a capping layer in which a low refractive index layer that contains the aromatic compound and a high refractive index layer are stacked, an organic EL device with improved light extraction efficiency can be realized. The invention has been proposed based on such findings, and specifically has the following constitution.
1) An aromatic compound represented by the following general formula (a) or the following general formula (b). 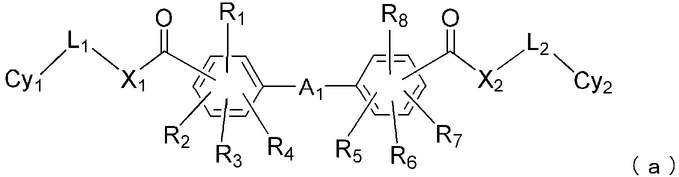 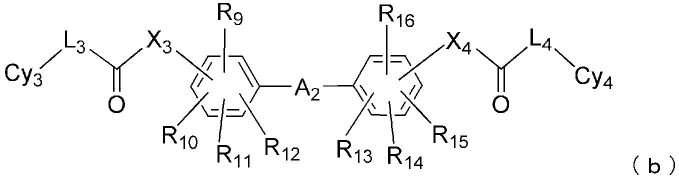
   In general formula (a) and the general formula (b), A₁ and A₂ each independently represent a substituted or unsubstituted alkanediyl group, a substituted or unsubstituted cycloalkanediyl group, or a substituted or unsubstituted fluorenediyl group,
   L₁ to L₄ each independently represent a single bond, -O-, -NH-, or a substituted or unsubstituted alkanediyl group,
   R₁ to R₁₆ each independently represent a hydrogen atom, a halogen atom, a substituted or unsubstituted alkyl group, a substituted or unsubstituted cycloalkyl group, a substituted or unsubstituted monovalent aromatic hydrocarbon group, or a substituted or unsubstituted monovalent aromatic heterocyclic group,
   Cy₁ to Cy₄ each independently represent a substituted or unsubstituted alkyl group, a substituted or unsubstituted cycloalkyl group, or a substituted or unsubstituted monovalent aromatic hydrocarbon group, and
   X₁ to X₄ each independently represent -O- or -NH-.
2) The aromatic compound according to 1), wherein R₁ to R₈ in the general formula (a) and R₉ to R₁₆ in the general formula (b) are each independently a hydrogen atom, a halogen atom, a substituted or unsubstituted alkyl group, or a substituted or unsubstituted cycloalkyl group.
3) The aromatic compound according to 1) or 2), wherein A₁ in the general formula (a) and A₂ in the general formula (b) are each independently a divalent group represented by the following formula (c), formula (d), formula (e), formula (f), or formula (g).

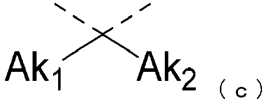

In the formula (c), Ak₁ and Ak₂ each independently represent a hydrogen atom, an unsubstituted alkyl group, or an unsubstituted haloalkyl group, and the dashed portions represent bonding sites.

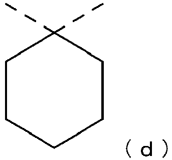

In the formula (d), the dashed portions represent bonding sites.

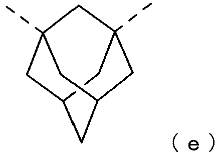

In the formula (e), the dashed portions represent bonding sites.

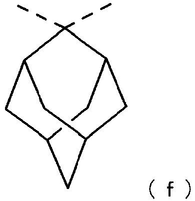

In the formula (f), the dashed portions represent bonding sites.

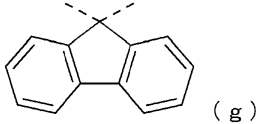

In the formula (g), the dashed portions represent bonding sites.

4) The aromatic compound according to any of 1) to 3), wherein L₁ and L₂ in the general formula (a) and L₃ and L₄ in the general formula (b) each independently represent a single bond, -O-, -NH-, a substituted or unsubstituted methylene group, a substituted or unsubstituted ethanediyl group, or a substituted or unsubstituted propanediyl group.

5) The aromatic compound according to any of 1) to 4), wherein Cy₁ and Cy₂ in the general formula (a) and Cy₃ and Cy₄ in the general formula (b) each independently represent a substituted or unsubstituted methyl group, a substituted or unsubstituted cyclohexyl group, a substituted or unsubstituted norbornyl group, a substituted or unsubstituted adamantyl group, or a substituted or unsubstituted phenyl group.

6) An organic EL device having at least an anode electrode, a hole transport layer, a light-emitting layer, an electron transport layer, a cathode electrode, and a capping layer in this order, in which the capping layer contains the aromatic compound of any of 1) to 5).

7) The organic EL device according to 6), in which the aromatic compound exhibits a refractive index of 1.70 or less at room temperature (25 ± 2°C) for light with a wavelength of 400 nm or more to 700 nm or less when vacuum-deposited on a silicon substrate at a film thickness of 80 nm to form a vapor-deposited film.

8) The organic EL device according to 6) or 7), in which the capping layer is a stacked or mixed layer including two or more compounds, and at least one compound in the capping layer is the aromatic compound.

9) An electronic component or an electronic device having a pair of electrodes and at least one organic layer, in which the organic layer contains an aromatic compound of any of 1) to 5).

### Advantageous Effects of Invention

The aromatic compound of the invention has excellent low refractive index properties. Accordingly, by combining a low refractive index layer formed with the compound with a high refractive index layer, an organic EL device with improved light emission efficiency can be realized due to the light interference effect.

In addition, the aromatic compound of the invention can be applied also in the field of electronic devices such as electrophotographic photoreceptors, image sensors, photoelectric conversion devices and solar cells, not limited to the use in organic EL devices.

### Brief Description of Drawings

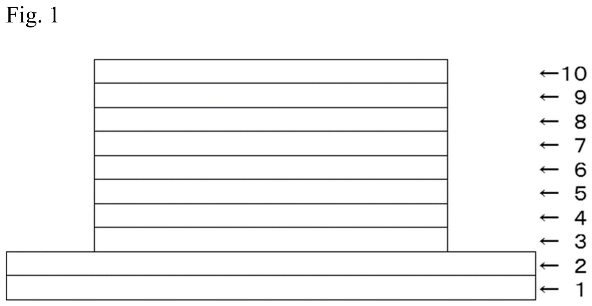

[Fig. 1] Fig. 1 is a figure showing an example of the configuration of the organic EL device of the invention.

### Description of Embodiments

The contents of the invention will be described in detail below. The constitutional elements can be described below with reference to representative embodiments and specific examples of the invention, but the invention is not limited to the embodiments and the examples. In the present description, a numerical range expressed using "to" means a range that includes the numerical values described before and after "to" as the lower limit and the upper limit. The hydrogen atom that is present in the molecule of the compound used in the invention is not particularly limited in isotope species, and for example, all the hydrogen atoms in the molecule may be ¹H, and all or a part of them may be ²H (deuterium D). In the present description, the term "substituted or unsubstituted" means that the group to which the term is attached may be an unsubstituted group (a group whose hydrogen atoms are not substituted with substituents) or that at least one hydrogen atom of the group may be substituted with a substituent.

In the present description, "transparent" means that the transmittance of visible light is 50% or more, e.g., 80% or more, e.g., 90% or more, e.g., 99% or more. The visible light transmittance can be measured with a UV/visible light spectrophotometer.

### <Aromatic Compound represented by general formula (a) or general formula (b)>

The compound of the invention is an aromatic compound represented by the following general formula (a) or the following general formula (b).

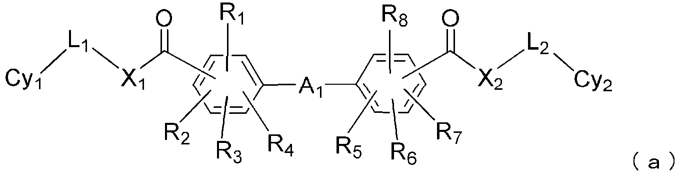

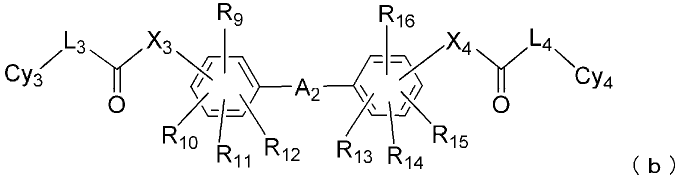

R₁ to R₈ in the general formula (a) and R₉ to R₁₆ in the general formula (b) each independently represent a hydrogen atom, a halogen atom, a substituted or unsubstituted alkyl group, a substituted or unsubstituted cycloalkyl group, a substituted or unsubstituted monovalent aromatic hydrocarbon group, or a substituted or unsubstituted monovalent aromatic heterocyclic group. R₁ to R₁₆ may be the same as or different from each other. Cy₁ and Cy₂ in the general formula (a) and Cy₃ and Cy₄ in the general formula (b) each independently represent a substituted or unsubstituted alkyl group, a substituted or unsubstituted cycloalkyl group, or a substituted or unsubstituted monovalent aromatic hydrocarbon group. Cy₁ to Cy₄ may be the same as or different from each other.

The "halogen atom" represented by R₁ to R₈ in the general formula (a) and R₉ to R₁₆ in the general formula (b) includes a fluorine atom, a chlorine atom, a bromine atom and an iodine atom, and a fluorine atom and a chlorine atom are preferred, and a fluorine atom is particularly preferred.

The "alkyl group" in the "substituted or unsubstituted alkyl group" represented by R₁ to R₈ and Cy₁ and Cy₂ in the general formula (a) and R₉ to R₁₆ and Cy₃ and Cy₄ in the general formula (b) may be linear or branched. The number of the carbon atoms of the alkyl group is, for example, 1 to 40, and may be 1 to 30, or 1 to 20, or maybe 1 to 10, or 1 to 6. The "alkyl group" specifically includes a methyl group, an ethyl group, an n-propyl group, an isopropyl group, an n-butyl group, an isobutyl group, an s-butyl group, and a t-butyl group; a methyl group, an isopropyl group, and a t-butyl group are preferred, and a t-butyl group is particularly preferred.

The "cycloalkyl group" in the "substituted or unsubstituted cycloalkyl group" represented by R₁ to R₈ and Cy₁ and Cy₂ in the general formula (a) and R₉ to R₁₆ and Cy₃ and Cy₄ in the general formula (b) may be a monocyclic cycloalkyl group, or may be a polycyclic cycloalkyl group. The polycyclic cycloalkyl group includes bicycloalkyl and tricycloalkyl groups. The number of the carbon atoms of the cycloalkyl group is, for example, 3 to 40, and may be 5 to 30, or 5 to 20, or 5 to 10. The "cycloalkyl group" specifically includes a cyclopentyl group, a cyclohexyl group, a cycloheptyl group, a cyclooctyl group, a norbornyl group, and an adamantyl group; and a cyclohexyl group, a cyclooctyl group, a norbornyl group, and an adamantyl group are preferred, and a norbornyl group and an adamantyl group are particularly preferred.

The "monovalent aromatic hydrocarbon group" in the "substituted or unsubstituted monovalent aromatic hydrocarbon group" represented by R₁ to R₈, Cy₁ and Cy₂ in the general formula (a) and R₉ to R₁₆, Cy₃ and Cy₄ in the general formula (b) may be a monocyclic aromatic hydrocarbon group, or may be a fused ring having two or more rings fused or a linked ring having two or more rings linked by a single bond. Here, each ring in the fused ring constituting the monovalent aromatic hydrocarbon group may be an aromatic hydrocarbon ring (e.g., benzene ring) or an aliphatic hydrocarbon ring, but the fused ring as a whole is an aromatic hydrocarbon ring. The number of the carbon atoms of the monovalent aromatic hydrocarbon group is, for example, 6 to 40, or may be 6 to 30, 6 to 20, or 6 to 14. Specifically, the "monovalent aromatic hydrocarbon group" includes phenyl, biphenylyl, 1-naphthyl, 2-naphthyl, 2-phenanthrenyl, 9-phenanthrenyl and fluorenyl groups; and phenyl, biphenylyl, 1-naphthyl, 2-naphthyl and fluorenyl groups are preferred, and a phenyl group is particularly preferred.

The "monovalent aromatic heterocyclic group" in the "substituted or unsubstituted monovalent aromatic heterocyclic group" represented by R₁ to R₈ in the general formula (a) and R₉ to R₁₆ in the general formula (b) may be a monocyclic aromatic heterocyclic group, or may include a fused ring having two or more rings fused. Here, the plurality of constituent rings of the fused ring that constitutes the monovalent aromatic heterocyclic group may be all hetero rings or may contain a hetero ring and a hydrocarbon ring (e.g., benzene ring). The constituent hetero and hydrocarbon rings may be aromatic or aliphatic rings, but the fused ring as a whole is an aromatic heterocyclic ring. The heteroatom that the monovalent aromatic heterocyclic group contains includes a nitrogen atom, a sulfur atom, and an oxygen atom. The number of the heteroatoms that the monovalent aromatic heterocyclic group contains may be one, or may be two or more. In the case where the monovalent aromatic heterocyclic group contains two or more heteroatoms, these heteroatoms may be the same or different. The number of the ring skeleton-constituting atoms of the monovalent aromatic heterocyclic group is, for example, 4 to 40, and may be 5 to 20, or 5 to 16, or 6 to 14. The number of the carbon atoms of the monovalent aromatic heterocyclic group is, for example, 3 to 35, and may be 3 to 30, or 2 to 20. Specifically, the "monovalent aromatic heterocyclic group" includes a pyridyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolynyl group; and a pyridyl group, a benzofuranyl group, a dibenzofuranyl group, a benzothienyl group, and a dibenzothienyl group are preferred, and a pyridyl group and a benzofuranyl group are particularly preferred.

The alkyl group, the cycloalkyl group, and the monovalent aromatic hydrocarbon group in R₁ to R₈ and Cy₁ and Cy₂ in the general formula (a) and R₉ to R₁₆ and Cy₃ and Cy₄ in the general formula (b) may be unsubstituted, or at least one hydrogen atom therein may be substituted with a substituent. The monovalent aromatic heterocyclic group in R₁ to R₈ in the general formula (a) and R₉ to R₁₆ in the general formula (b) may be unsubstituted, or at least one hydrogen atom therein may be substituted with a substituent. Specific examples of the "substituent" in the "substituted alkyl group", the "substituted cycloalkyl group", the "substituted aromatic hydrocarbon group", and the "substituted aromatic heterocyclic group" includes a cyano group, a nitro group; a halogen atom such as a fluorine atom, a chlorine atom, a bromine atom, and an iodine atom; a silyl group such as a trimethylsilyl group, and a triphenylsilyl group; a linear or branched alkyl group with 1 to 6 carbon atoms, such as a methyl group, an ethyl group, and a propyl group; a linear or branched haloalkyl group with 1 to 6 carbon atoms such as a trifluoromethyl group, a pentafluoroethyl group, a perfluoro-n-propyl group, a perfluoroisopropyl group, a perfluoro-n-butyl group, a perfluoro-s-butyl group, and a perfluoro-t-butyl group; a linear or branched alkoxy group with 1 to 6 carbon atoms such as a methoxy group, an ethoxy group group, and a propoxy group; an alkenyl group such as a vinyl group and an allyl group; an aryloxy group such as a phenyloxy group and a tolyloxy group; an aralkyloxy group such as a benzyloxy group and a phenethyloxy group; an aromatic hydrocarbon group such as a phenyl group, a biphenylyl group, a terphenylyl group, a naphthyl group, an anthracenyl group, a phenanthrenyl group, an indenyl group, a pyrenyl group, a perylenyl group, a fluoranthenyl group, a triphenylenyl group, a fluorenyl group, and a spirobifluorenyl group; an aromatic heterocyclic group such as a pyridyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group; and these substituents may be further substituted with the substituents exemplified above. In the case where these substituents are substituents of a benzene ring (including a benzene ring which is a constituent ring of a fused ring), the substituents and the substituted benzene ring, or a plurality of substituents substituted on the same benzene ring may bond to each other via a single bond, a substituted or unsubstituted methylene group, an oxygen atom or a sulfur atom to form a ring. For examples of the substituents of the methylene group, reference can be made to the description of the substituents of the above-mentioned "substituted alkyl group".

A₁ in the general formula (a) and A₂ in the general formula (b) each independently represent a substituted or unsubstituted alkanediyl group, a substituted or unsubstituted cycloalkanediyl group, or a substituted or unsubstituted fluorenediyl group. A₁ and A₂ may be the same as or different from each other. L₁ and L₂ in the general formula (a) and L₃ and L₄ in the general formula (b) each independently represent a single bond, -O-, -NH-, or a substituted or unsubstituted alkanediyl group. L₁ to L₄ may be the same as or different from each other. X₁ and X₂ in the general formula (a) and X₃ and X₄ in the general formula (b) each independently represent -O- or -NH-. X₁ to X₄ may be the same as or different from each other.

The "alkanediyl group" in the "substituted or unsubstituted alkanediyl group" represented by A₁, L₁ and L₂ in the general formula (a) and A₂, L₃ and L₄ in the general formula (b) is a divalent group obtained by removal of one hydrogen atom from an alkyl group. For the explanation of the "alkanediyl group", the description of the "alkyl group" in R₁ to R₈ above can be referred to by replacing the "alkyl group" with an "alkanediyl group". A specific example of the "alkanediyl group" can be a divalent group obtained by removal of one hydrogen atom from the specific group listed as an example of the "alkyl group" in R₁ to R₈ above.

The "cycloalkanediyl group" in the "substituted or unsubstituted cycloalkanediyl group" represented by A₁ in the general formula (a) and A₂ in the general formula (b) is a divalent group obtained by removal of one hydrogen atom from a cycloalkyl group. For the explanation of the "cycloalkanediyl group", the description of the"cycloalkyl group", the "bicycloalkyl group" and the "tricycloalkyl group" in R₁ to R₈ above can be referred to by replacing with a "cycloalkanediyl group", a "bicycloalkanediyl group" and a " tricycloalkanediyl group", respectively. A specific example of the "cycloalkanediyl group" can be a divalent group obtained by removal of one hydrogen atom from the specific group listed as an example of the "cycloalkyl group" in R₁ to R₈ above.

The alkanediyl group in A₁, L₁ and L₂ in the general formula (a) and A₂, L₃ and L₄ in the general formula (b) may be unsubstituted, or at least one hydrogen atom of the alkanediyl group may be substituted with a substituent. The cycloalkanediyl group and the fluorenediyl group in A₁ in the general formula (a) and A₂ in the general formula (b) may be unsubstituted, or at least one hydrogen atom of the cycloalkanediyl group and the fluorenediyl group may be substituted with a substituent. For examples of the "substituent" in the "substituted alkanediyl groups", the "substituted cycloalkanediyl groups", and the"substituted fluorenediyl groups", reference can be made to the description of the "substituent" in the "substituted alkyl groups", the "substituted cycloalkyl groups", and the "substituted aromatic hydrocarbon groups" represented by R₁ to R₈ above.

The "alkanediyl group" in A₁ in the general formula (a) and A₂ in the general formula (b) can be a group represented by the following formula (c).

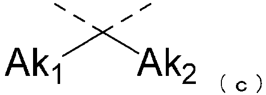

In the formula (c), Ak₁ and Ak₂ each independently represent a hydrogen atom, an unsubstituted alkyl group, or an unsubstituted haloalkyl group, and the dashed portions represent bonding sites (single bonds that each bond the carbon atom at the base of the dashed portion and the carbon atom of the benzene ring). Ak₁ and Ak₂ may be the same as or different from each other.

For the explanation of the "alkyl group" in the "unsubstituted alkyl group", represented by Ak₁ and Ak₂ in the formula (c), reference can be made to the description of the "alkyl group" in R₁ to R₈ above. Specific examples of the "alkyl group" include a methyl group, an ethyl group, an n-propyl group, an isopropyl group, an n-butyl group, an isobutyl group, an s-butyl group, and a t-butyl group; a methyl group, an isopropyl group, and a t-butyl group are preferred, and a methyl group is particularly preferred.

In the "unsubstituted haloalkyl group" represented by Ak₁ and Ak₂ in the formula (c), the "haloalkyl group" is one in which at least one hydrogen atom of the alkyl group is substituted with a halogen atom. For the explanation of the "alkyl group" and the "halogen atom" constituting the haloalkyl group, reference can be made to the description of the "alkyl group" and the "halogen atom" in R₁ to R₈ above. Specific examples of the "haloalkyl group" include a trifluoromethyl group, a 2,2,2-trifluoroethyl group, a pentafluoroethyl group, a perfluoro-n-propyl group, a perfluoroisopropyl group, a perfluoro-n-butyl group, and a perfluoro-t-butyl group; and a trifluoromethyl group, a perfluoroisopropyl group, and a perfluoro-t-butyl group are preferred, and a trifluoromethyl group is particularly preferred.

Examples of the "cycloalkanediyl group" in A₁ in the general formula (a) and A₂ in the general formula (b) include groups represented by any of the following formulas (d) to (f).

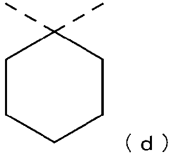

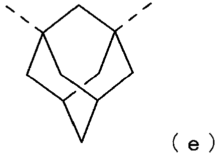

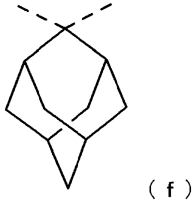

The "fluorenediyl group" in A₁ in the general formula (a) and A₂ in the general formula (b) can be a group represented by the following formula (g).

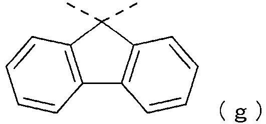

In formulas (d) to (g), the dashed portions represent bonding sites (single bonds that each bond the carbon atom at the base of the dashed portion and the carbon atom of the benzene ring).

In the aromatic compound represented by the general formula, Cy₁-L₁-X₁-C(=O)- and Cy₂-L₂-X₂-C(=O)- may bond at any position of the benzene ring (benzene ring constituting the ring skeleton) where the bond dangles. In one preferred aspect of the invention, the aromatic compound represented by the general formula (a) has a structure represented by the following general formula (a1).

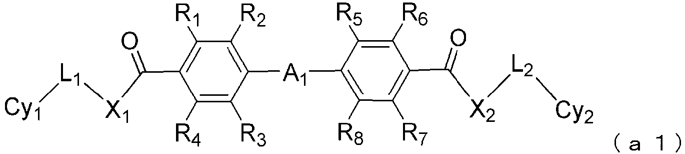

R₁ to R₈, Cy₁, Cy₂, A₁, L₁ and L₂ in the general formula (a1) are synonymous with R₁ to R₈, Cy₁, Cy₂, A₁, L₁ and L₂ in the general formula (a). For the description of R₁ to R₈, Cy₁, Cy₂, A₁, L₁ and L₂ in the general formula (a1), reference can be made to the corresponding description of the general formula (a).

In the aromatic compound represented by the general formula (b), Cy₃-L₃-C(=O)-X₃- and Cy₄-L₄-C(=O)-X₄- may bond at any position of the benzene ring (benzene ring constituting the ring skeleton) where the bond dangles. In one preferred aspect of the invention, the aromatic compound represented by the general formula (b) has a structure represented by the following general formula (b1).

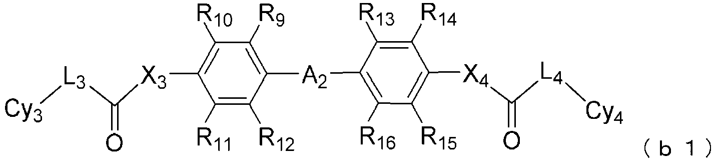

R₁ to R₁₆, Cy₃, Cy₄, A₂, L₃ and L₄ in the general formula (b1) are synonymous with R₁ to R₁₆, Cy₃, Cy₄, A₂, L₃ and L₄ in the general formula (b). For the description of R₁ to R₁₆, Cy₃, Cy₄, A₂, L₃ and L₄in the general formula (b1), reference can be made to the corresponding description of the general formula (b).

The general formula (a) and the general formula (a1) include a group where X₁ and X₂ are the same, a group where L₁ and L₂ are the same, a group where Cy₁ and Cy₂ are the same, a group where R₁ to R₄ are the same and R₅ to R₈ are the same, a group where R₁ to R₈ are the same, a group where X₁ and X₂ are the same and L₁ and L₂ are the same, a group where L₁ and L₂ are the same and Cy₁ and Cy₂ are the same, a group where X₁ and X₂ are the same, L₁ and L₂ are the same and Cy₁ and Cy₂ are the same, a group where X₁ and X₂ are the same, L₁ and L₂ are the same, Cy₁ and Cy₂ are the same, R₁ to R₄ are the same and R₅ to R₈ are the same, a group where X₁ and X₂ are the same, L₁ and L₂ are the same, Cy₁ and Cy₂ are the same and R₁ to R₈ are the same, a group where X₁ and X₂ are different, a group where L₁ and L₂ are different, a group where X₁ and X₂ are different and L₁ and L₂ are different, a group where X₁ and X₂ are O and L₁ and L₂ are single bonds, a group where X₁ and X₂ are NH and L₁ and L₂ are single bonds, a group where X₁ and X₂ are O and L₁ and L₂ each are an unsubstituted alkanediyl group, a group where X₁ and X₂ are NH and L₁ and L₂ each are an unsubstituted alkanediyl group, a group where X₁ and X₂ are O and L₁ and L₂ are NH, a group where X₁ and X₂ are NH and L₁ and L₂ are O, a group where X₁ and X₂ are NH and L₁ and L₂ are NH, a group where R₁ to R₈ are hydrogen atoms, a group where one or more of R₁ to R₈ are halogen atoms, a group where one or more of R₁ to R₈ are substituted or unsubstituted alkyl groups, a group where one or more of R₁ to R₈ are substituted or unsubstituted cycloalkyl groups, a group where one or more of R₁ to R₈ are substituted or unsubstituted monovalent aromatic hydrocarbon groups, and a group where one or more of R₁ to R₈ are substituted or unsubstituted monovalent aromatic heterocyclic groups.

The general formula (b) and the general formula (b1) include a group where X₃ and X₄ are the same, a group where L₃ and L₄ are the same, a group where Cy₃ and Cy₄ are the same, a group where R₉ to R₁₂ are the same, and R₁₃ to R₁₆ are the same, a group where R₉ to R₁₆ are the same, a group where X₃ and X₄ are the same and L₃ and L₄ are the same, a group where L₃ and L₄ are the same and Cy₃ and Cy₄ are the same, a group where X₃ and X₄ are the same, L₃ and L₄ are the same and Cy₃ and Cy₄ are the same, a group where X₃ and X₄ are the same, L₃ and L₄ are the same, Cy₃ and Cy₄ are the same, R₉ to R₁₂ are the same and R₁₃ to R₁₆ are the same, a group where X₃ and X₄ are the same, L₃ and L₄ are the same, Cy₃ and Cy₄ are the same and R₉ to R₁₆ are the same, a group where X₃ and X₄ are different, a group where L₃ and L₄ are different, a group where X₃ and X₄ are different and L₃ and L₄ are different, a group where X₃ and X₄ are O and L₃ and L₄ are single bonds, a group where X₃ and X₄ are NH and L₃ and L₄ are single bonds, a group where X₃ and X₄ are O and L₃ and L₄ each are an unsubstituted alkanediyl group, a group where X₃ and X₄ are NH and L₃ and L₄ each are an unsubstituted alkanediyl group, a group where X₃ and X₄ are O and L₃ and L₄ are NH, a group where X₃ and X₄ are O and L₃ and L₄ are O, a group where X₃ and X₄ are NH and L₃ and L₄ are O, a group where X₃ and X₄ are NH and L₃ and L₄ are NH, a group where R₉ to R₁₆ are hydrogen atoms, a group where one or more of R₉ to R₁₆ are halogen atoms, a group where one or more of R₉ to R₁₆ are substituted or unsubstituted alkyl groups, a group where one or more of R₉ to R₁₆ are substituted or unsubstituted cycloalkyl groups, a group where one or more of R₉ to R₁₆ are substituted or unsubstituted monovalent aromatic hydrocarbon groups, and a group where one or more of R₉ to R₁₆ are substituted or unsubstituted monovalent aromatic heterocyclic groups.

Compound groups of the invention include Compound Group 1 represented by the general formula (a). Compound Group 1 includes Compound Group 1a, wherein A₁ is a substituted or unsubstituted alkanediyl group and Cy₁ and Cy₂ each are an substituted or unsubstituted cycloalkyl group; Compound Group 1b, wherein A₁ is a substituted or unsubstituted cycloalkanediyl group and Cy₁ and Cy₂ each are a substituted or unsubstituted cycloalkyl group; Compound Group 1c, wherein A₁ is a substituted or unsubstituted fluorenediyl group and Cy₁ and Cy₂ each are an substituted or unsubstituted cycloalkyl group; Compound Group 1d, wherein A₁ is a substituted or unsubstituted alkanediyl group and Cy₁ and Cy₂ each are a substituted or unsubstituted monovalent aromatic hydrocarbon group; Compound Group 1e, wherein A₁ is a substituted or unsubstituted cycloalkanediyl group and Cy₁ and Cy₂ each are substituted or unsubstituted monovalent aromatic hydrocarbon group; Compound Group 1f, wherein A₁ is a substituted or unsubstituted fluorenediyl group and Cy₁ and Cy₂ each are a substituted or unsubstituted monovalent aromatic hydrocarbon group; Compound Group 1g, wherein A₁ is a substituted or unsubstituted alkanediyl group and Cy₁ and Cy₂ each are a substituted or unsubstituted alkyl group; Compound Group 1h, wherein A₁ is a substituted or unsubstituted cycloalkanediyl group and Cy₁ and Cy₂ each are a substituted or unsubstituted alkyl group; and Compound Group 1i, wherein A₁ is a substituted or unsubstituted fluorenediyl group and Cy₁ and Cy₂ each are a substituted or unsubstituted alkyl group.

Compound Groups 1a to 1i can each further satisfy at least one of the following additional requirements. One additional requirement is that A₁ is an unsubstituted alkanediyl group, an unsubstituted cycloalkanediyl group, or an unsubstituted fluorenediyl group. One additional requirement is that Cy₁ and Cy₂ each are an unsubstituted cycloalkyl group, an unsubstituted monovalent aromatic hydrocarbon group, or an unsubstituted alkyl group. One additional requirement is that Cy₁ and Cy₂ are the same. One additional requirement is the requirement described for any one of the above-mentioned groups listed as being included in general formula (a) and the general formula (a1).

Compound groups of the invention include Compound Group 2 represented by the general formula (b). Compound Group 2 includes Compound Group 2a, wherein A₂ is a substituted or unsubstituted alkanediyl group and Cy₃ and Cy₄ each are an substituted or unsubstituted cycloalkyl group; Compound Group 2b, wherein A₂ is a substituted or unsubstituted cycloalkanediyl group and Cy₃ and Cy₄ each are a substituted or unsubstituted cycloalkyl group; Compound Group 2c, wherein A₂ is a substituted or unsubstituted fluorenediyl group and Cy₃ and Cy₄ each are an substituted or unsubstituted cycloalkyl group; Compound Group 2d, wherein A₂ is a substituted or unsubstituted alkanediyl group and Cy₃ and Cy₄ each are a substituted or unsubstituted monovalent aromatic hydrocarbon group; Compound Group 2e, wherein A₂ is a substituted or unsubstituted cycloalkanediyl group and Cy₃ and Cy₄ each are substituted or unsubstituted monovalent aromatic hydrocarbon group; Compound Group 2f, wherein A₂ is a substituted or unsubstituted fluorenediyl group and Cy₃ and Cy₄ each are a substituted or unsubstituted monovalent aromatic hydrocarbon group; Compound Group 2g, wherein A₂ is a substituted or unsubstituted alkanediyl group and Cy₃ and Cy₄ each are a substituted or unsubstituted alkyl group; Compound Group 2h, wherein A₂ is a substituted or unsubstituted cycloalkanediyl group and Cy₃ and Cy₄ each are a substituted or unsubstituted alkyl group; and Compound Group 2i, wherein A₂ is a substituted or unsubstituted fluorenediyl group and Cy₃ and Cy₄ each are a substituted or unsubstituted alkyl group.

Compound Groups 2a to 2i can each further satisfy at least one of the following additional requirements. One additional requirement is that A₂ is an unsubstituted alkanediyl group, an unsubstituted cycloalkanediyl group, or an unsubstituted fluorenediyl group. One additional requirement is that Cy₃ and Cy₄ each are unsubstituted cycloalkyl group, an unsubstituted monovalent aromatic hydrocarbon group, or an unsubstituted alkyl group. One additional requirement is that Cy₃ and Cy₄ are the same. One additional requirement is the requirement described for any one of the above-mentioned groups listed as being included in general formula (b) and the general formula (b1).

Hereinafter, specific examples of the aromatic compounds represented by the general formula (a) and the general formula (b) will be given. However, the aromatic compounds represented by the general formula (a) and the general formula (b) that can be used in the invention should not be construed as being limited by these specific examples.

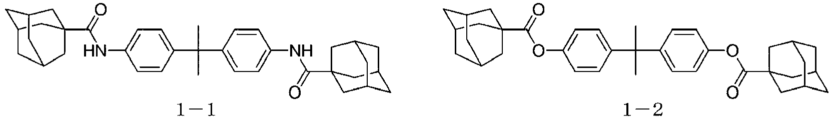

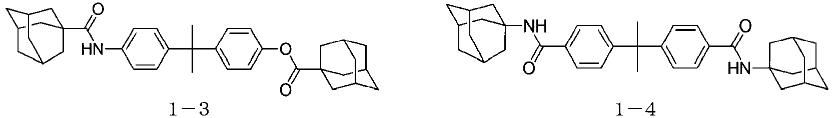

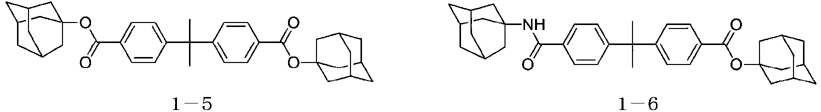

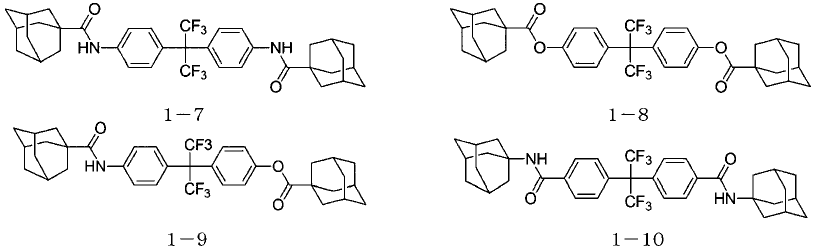

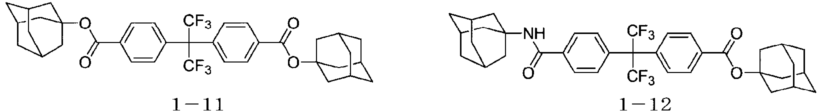

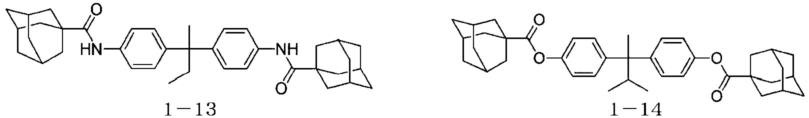

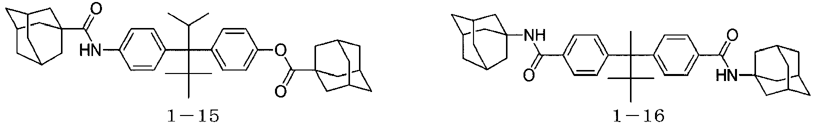

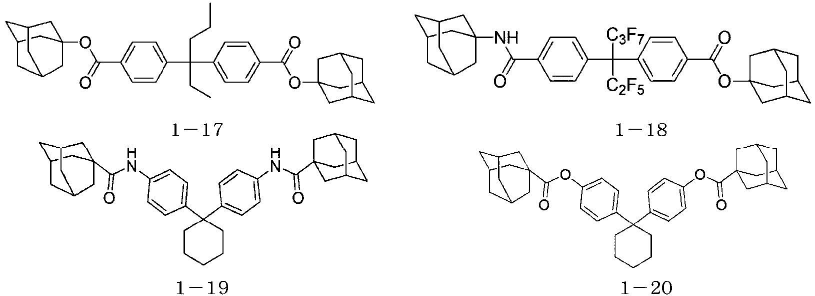

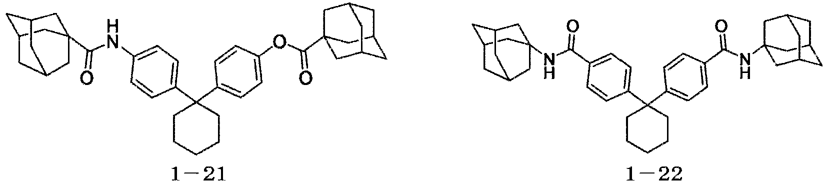

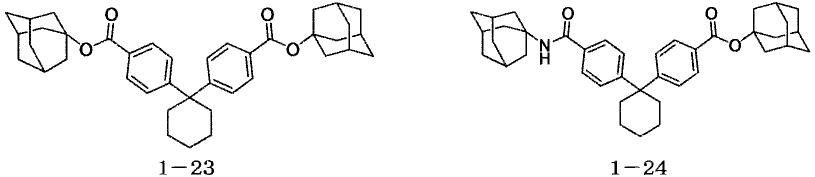

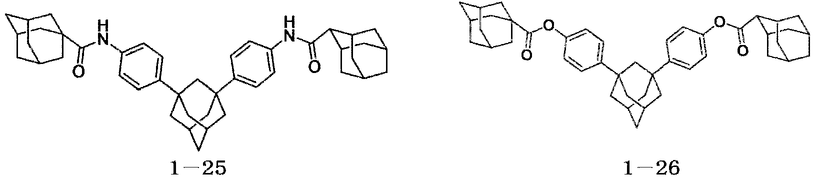

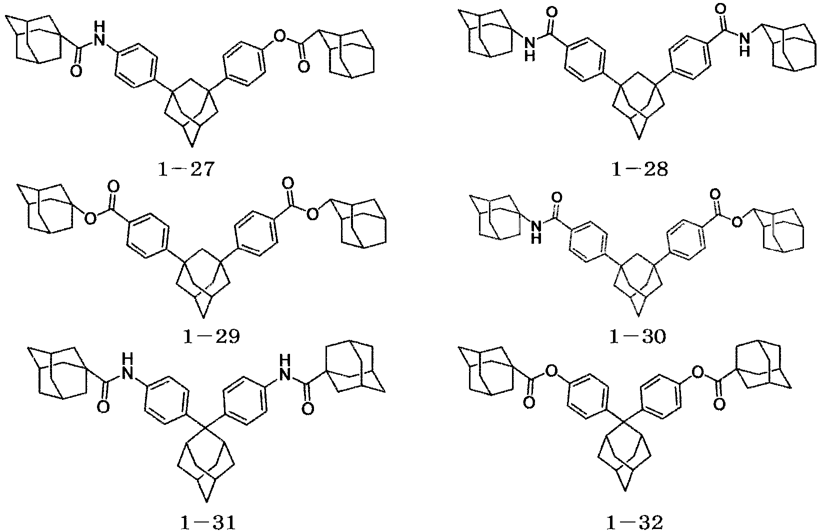

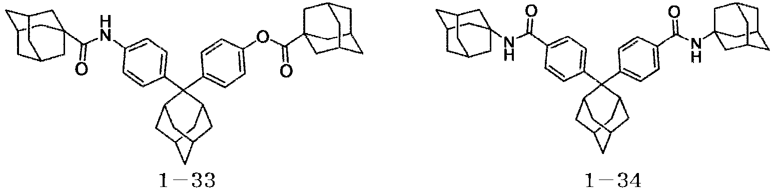

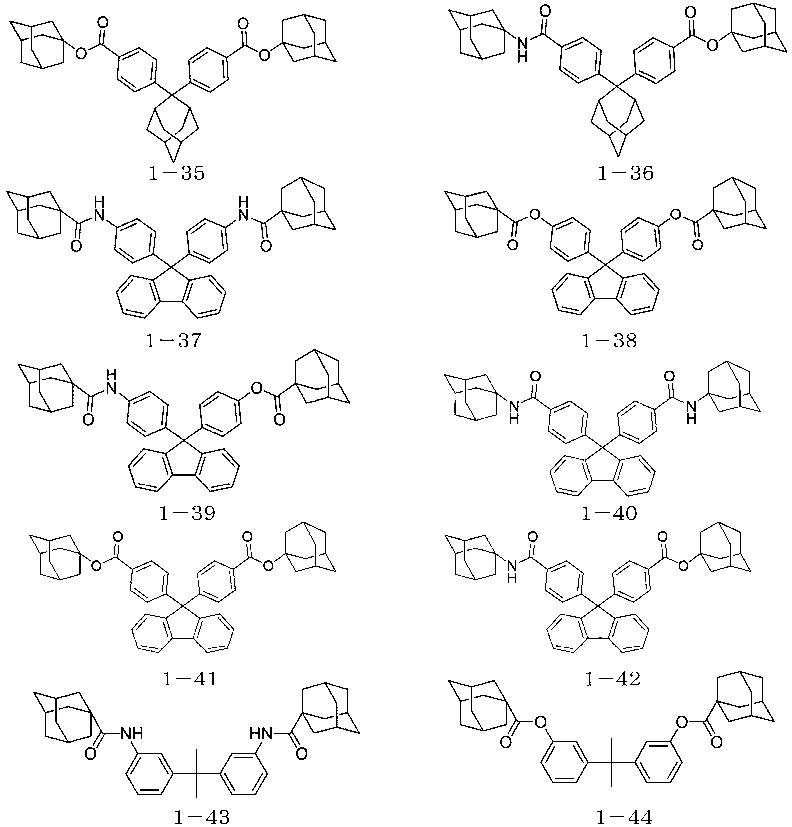

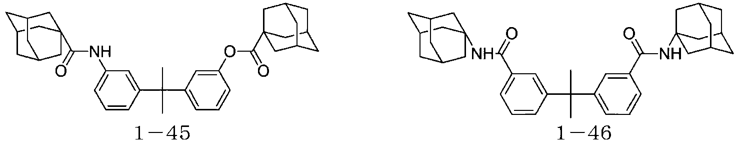

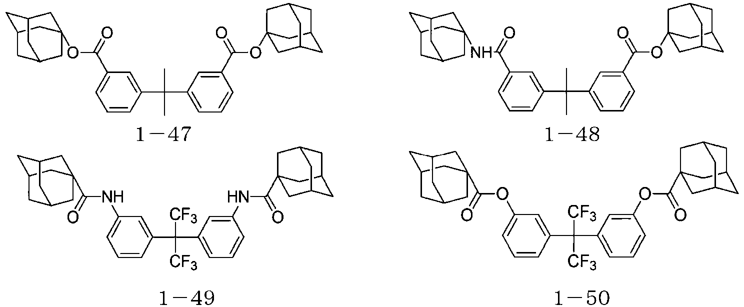

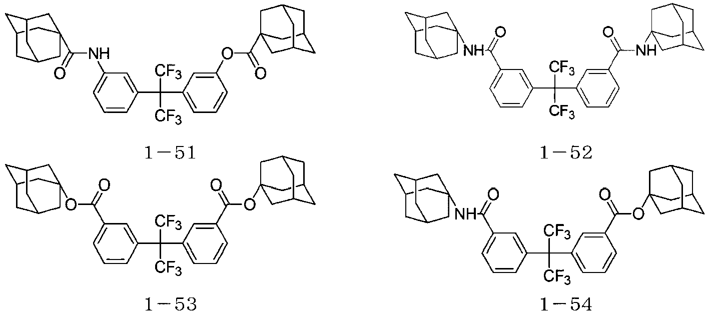

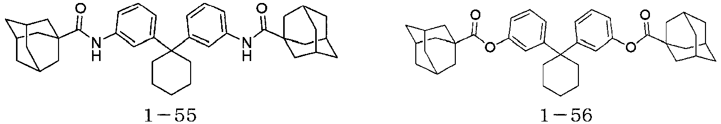

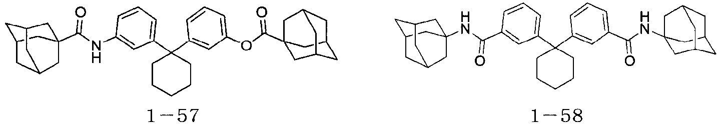

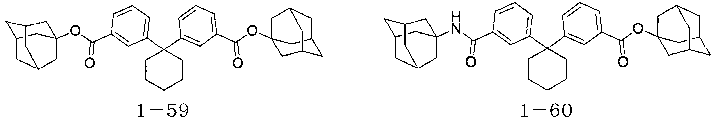

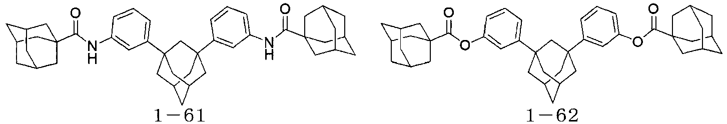

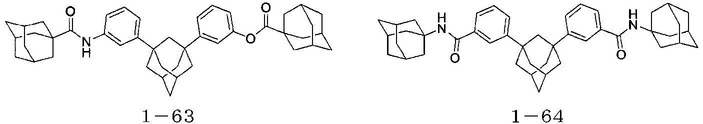

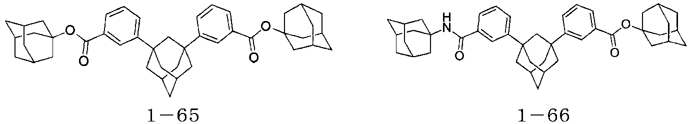

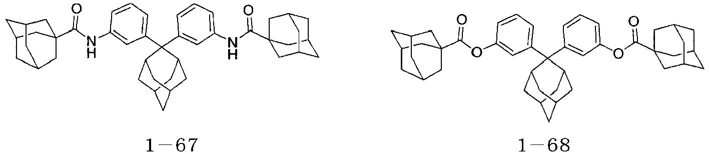

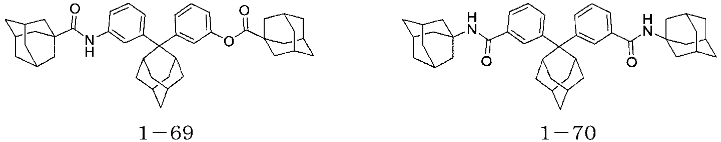

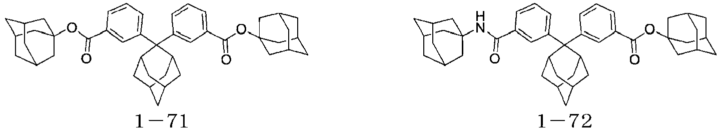

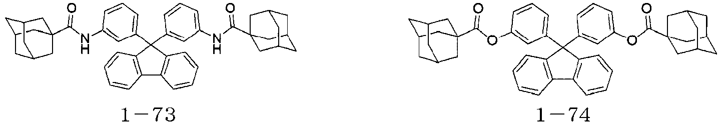

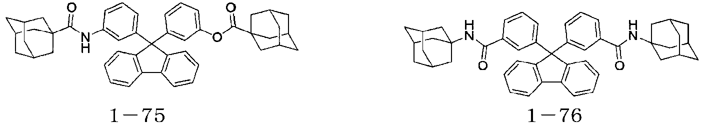

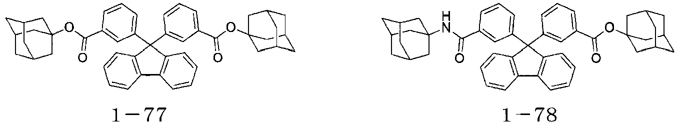

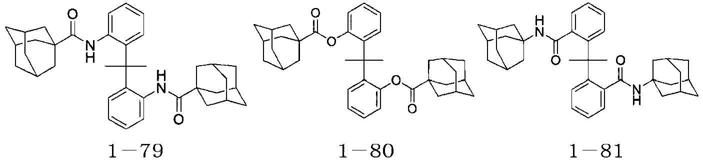

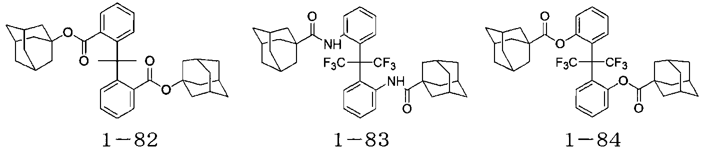

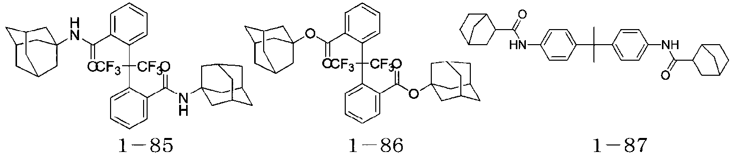

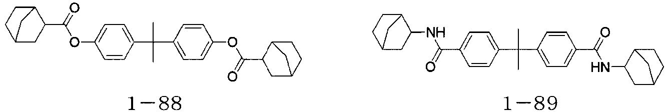

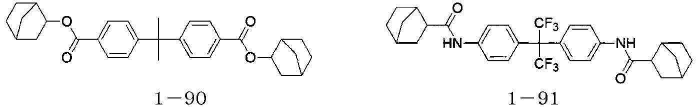

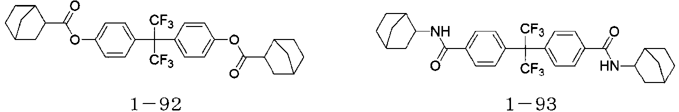

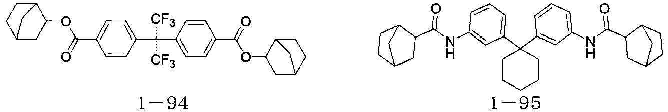

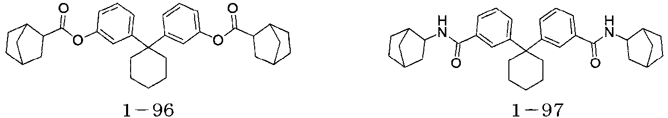

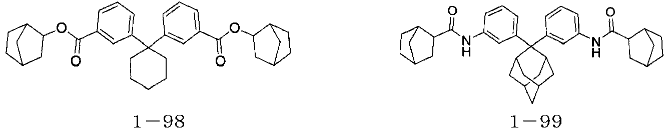

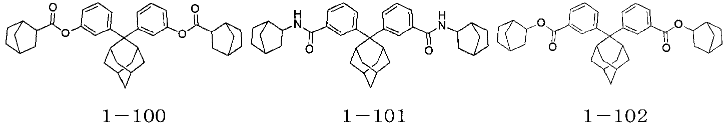

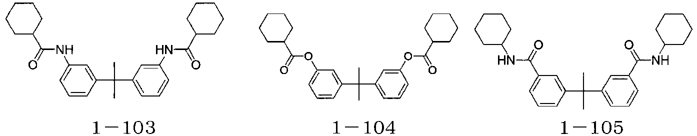

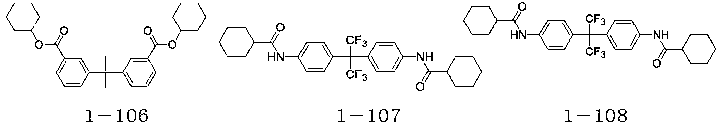

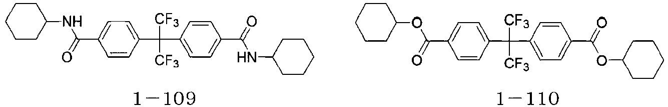

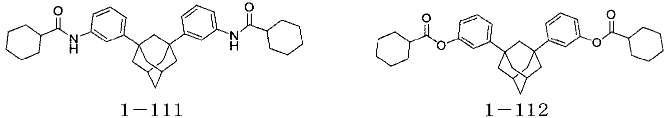

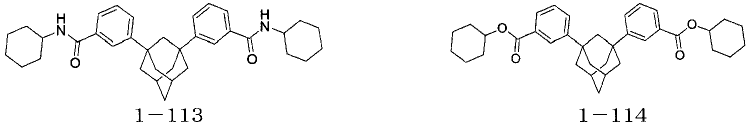

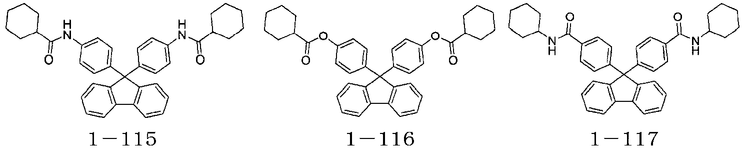

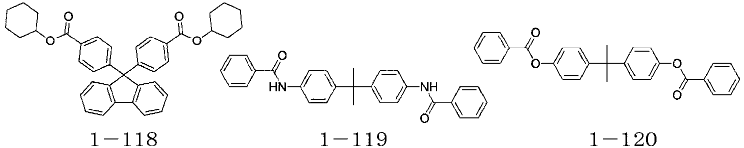

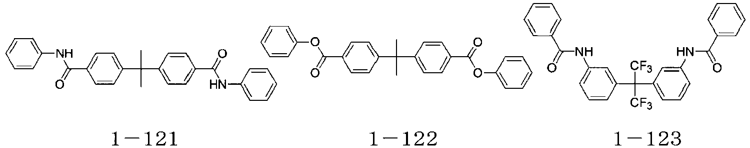

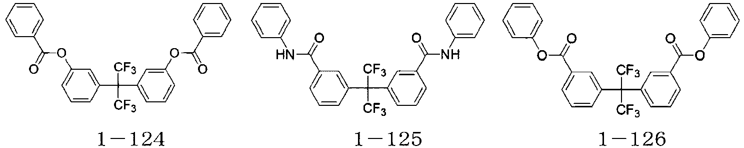

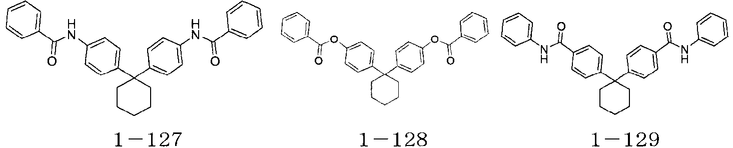

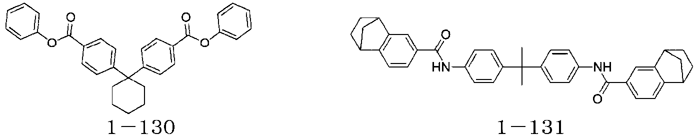

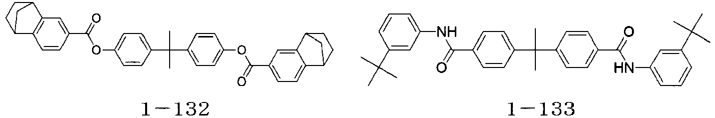

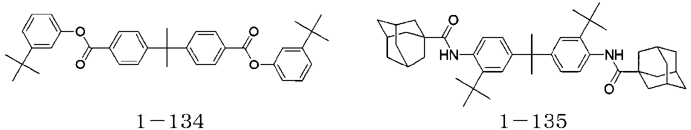

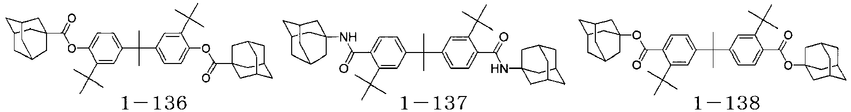

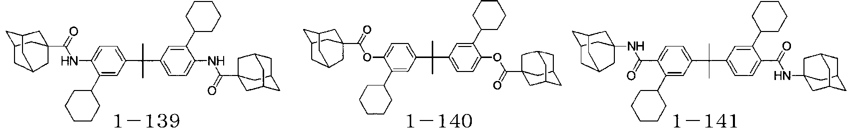

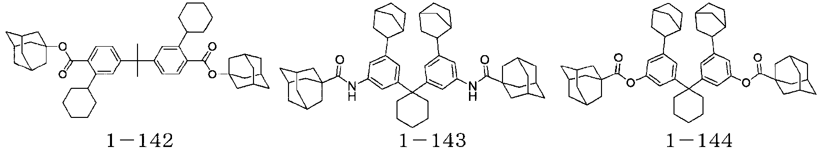

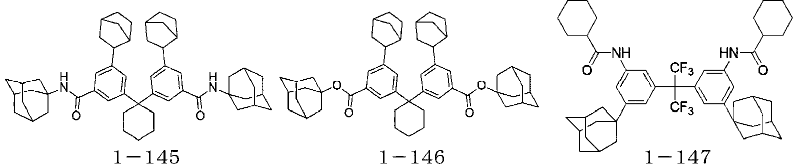

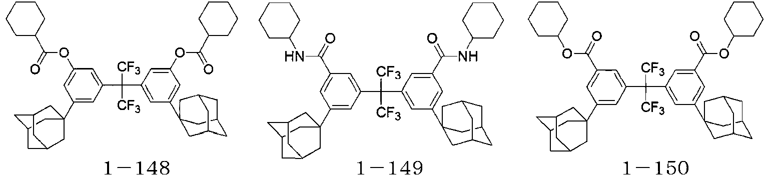

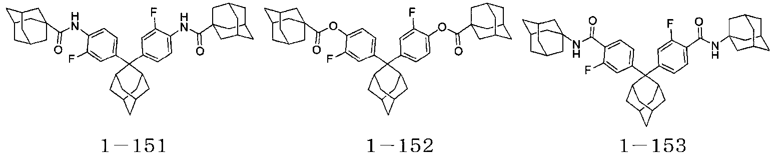

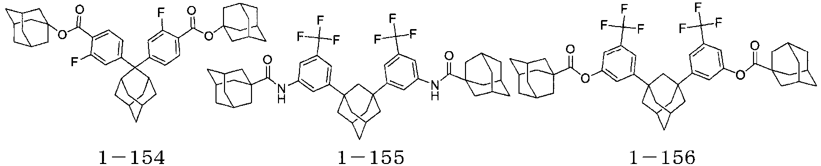

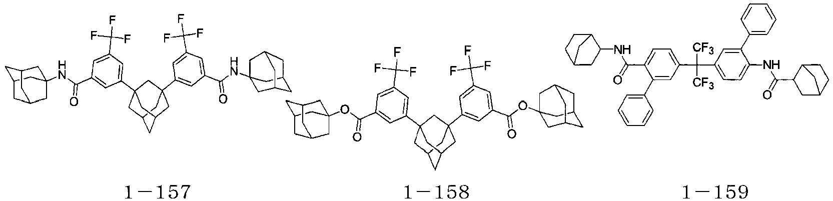

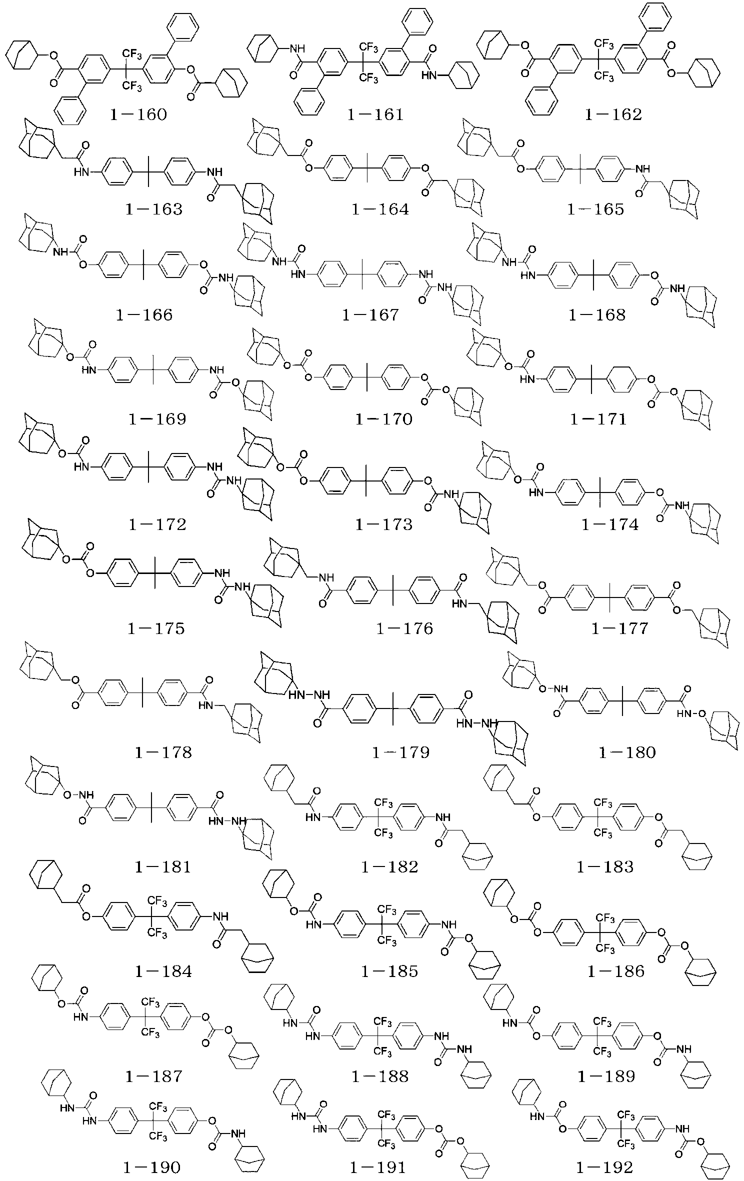

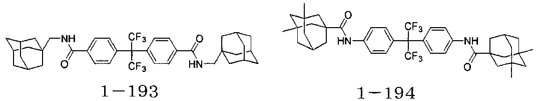

### <Synthesis Method for aromatic compound represented by general formula (a) or general formula (b)>

The aromatic compound represented by the general formula (a) or the general formula (b) is a novel compound. The aromatic compound represented by the general formula (a) or the general formula (b) can be synthesized using known reactions, for example, the reaction of an acid chloride with an amine or an acid chloride with a hydroxyl compound. For details of the synthesis method, Synthesis Examples described later can be referred to.

The production method for the aromatic compound represented by the general formula (a) or the general formula (b) is not specifically limited. For example, the aromatic compound can be purified by column chromatography, solvent recrystallization or crystallization, sublimation purification, and other known methods used to purify organic compounds. The compound can be identified by NMR analysis or mass spectrometry.

Preferably, the melting point, the glass transition point (Tg) and the refractive index are measured as the physical properties of the aromatic compound represented by the general formula (a) or the general formula (b) of the invention. The melting point is an indicator of deposition properties, the glass transition point (Tg) is an indicator of the stability in a thin film state, and the refractive index is an indicator regarding the improvement of light extraction efficiency.

The melting point and the glass transition point (Tg) of the aromatic compound of the invention can be measured, for example, with a high-sensitivity differential scanning calorimeter (DSC 3100SA, manufactured by Bruker AXS) using a powder.

The refractive index and the extinction coefficient of the aromatic compound of the invention can be measured using a spectrometer (F10-RT-UV, manufactured by Filmetrics, Inc.) with an 80-nm thin film formed on a silicon substrate.

The aromatic compound represented by the general formula (a) or the general formula (b) of the invention is characterized by having a low refractive index. Therefore, when a capping layer formed by stacking a first capping layer containing the aromatic compound (low refractive index layer) and a second capping layer having a refractive index higher than that of the first capping layer (high refractive index layer) is placed at a position outside the electrode on the light extraction side of an organic EL device (opposite side of the electrode from the light-emitting layer) so that the first capping layer is on the electrode side, then the light extraction efficiency is improved by the effective light interference effect in the capping layer, and high light emission efficiency can be achieved. The aromatic compound represented by the general formula (a) or the general formula (b) has a melting point suitable for vapor deposition and has a high glass transition temperature to secure a stable thin film state.

Here, the "capping layer" in this specification means a layer placed on an opposite side (outside) of at least one of a pair of electrodes to the light-emitting layer in an organic EL device in which the light-emitting layer is arranged between the pair of electrodes. The capping layer containing the aromatic compound represented by the general formula (a) or the general formula (b) may be placed only on the outside of one of the pair of electrodes or on the outside of both electrodes. An organic layer such as a charge transport layer may be arranged between the light-emitting layer and each electrode of the organic EL device to which the capping layer is applied.

### <Organic Electroluminescent Device>

Next, the organic electroluminescent device (organic EL device) of the invention will be described. The organic EL device of the invention has at least an anode electrode, a hole transport layer, a light-emitting layer, an electron transport layer, a cathode electrode, and a capping layer in this order, in which the capping layer contains the aromatic compound represented by the general formula (a) or the general formula (b).

For the description of the aromatic compound represented by the general formula (a) or the general formula (b), reference can be made to the description in the above section of "Aromatic Compound Represented by General Formula (a) or General Formula (b)".

In one preferred aspect of the invention, the capping layer is formed by stacking the first capping layer (low refractive index layer) containing the aromatic compound represented by the general formula (a) or the general formula (b) and the second capping layer (high refractive index layer) with a higher refractive index than the first capping layer. In this configuration, the light extraction efficiency of the organic EL device is improved by the second capping layer with a high refractive index, and the high reflectivity at the interface between the second capping layer and the first capping layer causes a light interference effect, which further enhances the increasing effect of the second capping layer on the light extraction efficiency.

Preferably, the capping layers is so arranged that, of the first capping layer and the second capping layer, the first capping layer is on the electrode side (the second capping layer is on the outside). In one more preferred aspect of the invention, the organic EL device has at least an anode electrode, a hole transport layer, a light-emitting layer, an electron transport layer, a cathode electrode, a first capping layer and a second capping layer in this order.

For example, the configuration of the organic EL device of the invention, in the case of a light-emitting device with a top emission structure, includes an anode, a hole transport layer, a light-emitting layer, an electron transport layer, a cathode, and a capping layer, sequentially on a substrate made of glass, and also has a hole injection layer between the anode and the hole transport layer, has an electron-blocking layer between the hole transport layer and the light-emitting layer, has a hole blocking layer between the light-emitting layer and the electron transport layer, or has an electron injection layer between the electron transport layer and the cathode. In these multilayer structures, some organic layers can be omitted or one organic layer can play multiple roles, for example, an organic layer may serve as a hole injection layer and a hole transport layer, an organic layer may serve as a hole transport layer and an electron-blocking layer, an organic layer may serve as a hole-blocking layer and an electron transport layer, or an organic layer may serve as an electron transport layer and an electron injection layer. It is also possible to provide a stack structure with two or more organic layers having the same function, such as a stack structure with two hole transport layers, a stack structure with two light-emitting layers, a stack structure with two electron transport layers, and a stack structure with two capping layers.

The total thickness of the layers of the organic EL device is preferably 200 nm to 750 nm or so, more preferably 350 nm to 600 nm or so. The thickness of the capping layer is, for example, preferably 30 nm to 120 nm, more preferably 40 nm to 80 nm. In that case, good light extraction efficiency is attained. The thickness of the capping layer can be appropriately changed according to the type of light-emitting material used in the light-emitting device, and the thickness of the other layers than the capping layer of the organic EL device.

In the following, the constituent members and layers of the organic EL device will be described.

### [Anode Electrode]

Electrode materials with a large work function such as ITO (indium tin oxide) and gold are used as materials for the anode of the organic EL device of the invention.

### [Hole Injection Layer]

As a material for the hole injection layer of the organic EL device of the invention, arylamine compounds having three or more triphenylamine structures in the molecule, in which these triphenylamine structures are linked via a single bond or a divalent group containing no heteroatom, can be listed. As arylamine compounds, for example, materials such as starburst triphenylamine derivatives and various triphenylamine tetramers can be listed. Porphyrin compounds typified by copper phthalocyanine, acceptor heterocyclic compounds such as hexacyanoazatriphenylene, and coating-type polymer materials can also be used as hole injection layer materials. The hole injection layer may be a single layer of one of these hole injection materials deposited alone, or may be a mixed layer formed from two or more materials in admixture. The hole injection layer may be a single-layer structure; a stack structure of layers each formed from a single material; a stack structure of layers each formed from two or more materials in admixture; or a stack structure including a layer formed from a single material and a layer formed from two or more materials in admixture. These materials can be formed into thin films by known methods such as vapor deposition, and in addition, spin-coating, or ink-jetting.

### [Hole Transport Layer]

Benzidine derivatives such as N,N'-diphenyl-N,N'-di(m-tolyl)benzidine (hereinafter abbreviated as TPD), N,N'-diphenyl-N,N'-di(a-naphthyl)benzidine(hereinafter abbreviated as NPD) and N,N,N',N'-tetrabiphenylylbenzidine, and also 1,1-bis[4-(di-4-tolylamino)phenyl]cyclohexane (hereinafter abbreviated as TAPC) can be used for the hole transport layer of the organic EL device of the invention. Arylamine compounds, especially those having two triphenylamine structures in the molecule, in which these triphenylamine structures are linked via a single bond or a divalent group containing no heteroatom, such as N, N, N', N'-tetrabiphenylylbenzidine are preferably used. Arylamine compounds, especially those having three triphenylamine structures in the molecule, in which these triphenylamine structures are linked via a single bond or a divalent group containing no heteroatom, such as various triphenylamine trimers and tetramers are preferably used. The hole transport layer may be a single layer formed from one of these hole transport materials alone, or may be a mixed layer formed from two or more materials in admixture. The hole transport layer may be a single-layer structure; a stack structure of layers each formed from a single material or layers each formed from two or more materials in admixture; or a stack structure including a layer formed from a single material and a layer formed from two or more materials in admixture. Coating-type polymer materials such as poly(3,4-ethylenedioxythiophene) (hereinafter abbreviated as PEDOT)/poly(styrenesulfonate) (hereinafter abbreviated as PSS) can also be used as the hole injection/transport layer. These materials can be formed into thin films by known methods such as vapor deposition, and in addition, spin-coating, or ink-jetting.

In addition, as materials for the hole injection layer or the hole transport layer, further usable are those formed by doping a p-type dopant such as tris-bromophenylamine hexachloroantimony or a radialene derivative into the materials normally used for these layers, and also polymer compounds containing a benzidine derivative structure as a substructure such as TPD.

### [Electron-Blocking Layer]

The organic EL device of the invention may have an electron-blocking layer between the light-emitting layer and the hole transport layer. As materials for the electron-blocking layer, compounds having electron blocking effect can be used, such as carbazole derivatives, for example, 4,4',4"-tri(N-carbazolyl)triphenylamine (hereinafter abbreviated as TCTA), 9,9-bis[4-(carbazol-9-yl)phenyl]fluorene, 1,3-bis(carbazol-9-yl)benzene (hereinafter abbreviated as mCP), and 2,2-bis(4-carbazol-9-ylphenyl)adamantane (hereinafter abbreviated as Ad-Cz); and compounds having a triphenylsilyl group and a triarylamine structure, as typified by 9-[4-(carbazol-9-yl)phenyl]-9-[4-(triphenylsilyl)phenyl]-9H-fluorenone. The electron-blocking layer may be a single layer formed from one of these electron-blocking materials alone, or may be a mixed layer formed from two or more materials in admixture. The electron-blocking layer may be a single-layer structure; a stack structure of layers each formed from a single material or layers each formed from two or more materials in admixture, or a stack structure including a layer formed from a single material and a layer formed from two or more materials in admixture. These materials can be formed into thin films by known methods such as vapor deposition, and in addition, spin-coating, or ink-jetting.

### [Light-Emitting Layer]

As materials for the light-emitting layer of the organic EL device of the invention, light-emitting materials, such as metal complexes of quinolinol derivatives typified by tris(8-quinolylato)aluminum (Alq₃), and other various metal complexes, anthracene derivatives, bis-styrylbenzene derivatives, pyrene derivatives, oxazole derivatives and poly(para-phenylene-vinylene) derivatives can be used. Also, the light-emitting layer may be formed of a host material and a dopant material, and as the host material, anthracene derivatives are preferably used, and in addition to the above-mentioned light-emitting materials, also usable are heterocyclic compounds with an indole ring as a partial structure of a fused ring therein, heterocyclic compounds with a carbazole ring as a partial structure of a fused ring therein, carbazole derivatives, thiazole derivatives, benzimidazole derivatives, and polydialkylfluorene derivatives. Quinacridone, coumarin, rubrene, perylene and their derivatives, benzopyran derivatives, rhodamine derivatives, and aminostyryl derivatives can be used as dopant materials, and green light-emitting materials are particularly preferable. The light-emitting layer may be a single layer formed from one of the above-mentioned light-emitting materials alone, or may be a mixed layer formed from two or more materials (e.g., two or more light-emitting materials, or one or more host materials and one or more dopant materials) in admixture. The light-emitting layer may be a single-layer structure; a stack structure of layers each formed from a single material or layers each formed from two or more materials in admixture; or a stack structure including a layer formed from a single material and a layer formed from two or more materials in admixture.

Phosphorescent light emitters can also be used as the light-emitting material. Phosphorescent light emitters of metal complexes of iridium or platinum can be used as the phosphorescent light emitters. For example, green phosphorescent light emitters such as Ir(ppy)₃ (tris(2-phenylpyridinato)iridium(III)), blue phosphorescent light emitters such as FIrpic (bis[2-(4,6-difluorophenyl)pyridinato-C2,N](picolinato)iridium(III)) and FIr6 (bis(2,4-difluorophenylpyridinato)tetrakis(1-pyrazolyl)borate iridium(III)), and red phosphorescent light emitters such as Btp₂Ir(acac) (bis(2-benzo[b]thiophen-2-yl-pyridine)(acetylacetonato)iridium(III)) can be used, and green phosphorescent emitters are particularly preferable. The light-emitting layer may be composed solely of these phosphorescent light emitters, or may contain a host material and a phosphorescent light emitter (e.g., a co-deposited film of a host material and a phosphorescent light emitter). Carbazole derivatives such as 4,4'-di(N-carbazolyl)biphenyl (hereafter abbreviated as CBP), TCTA and mCP can be used as hole injection and transporting host materials. As electron transporting host materials, p-bis(triphenylsilyl)benzene (hereinafter abbreviated as UGH2) and 2,2',2"-(1,3,5-phenylene)-tris(1-phenyl-1H-benzimidazole) (hereinafter abbreviated as TPBI) can be used to produce high-performance organic EL devices.

The amount of the phosphorescent light emitter doped into the host material is preferably in a range of 1 to 30% by weight of the amount of the light-emitting layer to avoid concentration quenching.

Delayed fluorescence light emitters can also be used as the light-emitting material. These materials can be formed into thin films by known methods such as vapor deposition, and in addition, spin-coating, or ink-jetting.

### [Hole Blocking Layer]

The organic EL device of the invention may have a hole-blocking layer between the light-emitting layer and the electron transport layer. As the material for the hole-blocking layer, usable are hole-blocking compounds, e.g., phenanthroline derivatives such as bathocuproine (hereinafter abbreviated as BCP), metal complexes of quinolinol derivatives such as aluminum(III) bis(2-methyl-8-quinolinato)-4-phenylphenolate (hereafter abbreviated as BAIq), various rare earth complexes, triazole derivatives, triazine derivatives, pyrimidine derivatives, oxadiazole derivatives and benzazole derivatives. These materials may also serve as materials for the electron transport layer. The hole-blocking layer may be a single layer formed from one of these electron-blocking materials alone, or may be a mixed layer formed from two or more materials in admixture. The hole-blocking layer may be a single-layer structure; a stack structure of layers each formed from a single material or layers each formed from two or more materials in admixture; or a stack structure including a layer formed from a single material and a layer formed from two or more materials in admixture. These materials can be formed into thin films by known methods such as vapor deposition, and in addition, spin-coating, or ink-jetting.

### [Electron Transport Layer]

As materials for the electron transport layer of the organic EL device of the invention, usable are metal complexes of quinolinol derivatives such as typically Alq₃ and BAlq, and other various metal complexes, triazole derivatives, triazine derivatives, pyrimidine derivatives, oxadiazole derivatives, pyridine derivatives, benzimidazole derivatives, benzazole derivatives, thiadiazole derivatives, anthracene derivatives, carbodiimide derivatives, quinoxaline derivatives, pyridoindole derivatives, phenanthroline derivatives and silole derivatives. The electron transport layer may be a single layer formed from one of these electron transport materials alone, or may be a mixed layer formed from two or more materials in admixture. The electron transport layer may be a single-layer structure; a stack structure of layers each formed from a single material or layers each formed from two or more materials in admixture; or a stack structure including a layer formed from a single material and a layer formed from two or more materials in admixture. These materials can be formed into thin films by known methods such as vapor deposition, and in addition, spin-coating, or ink-jetting.

### [Electron Injection Layer]

As materials for the electron injection layer of the organic EL device of the invention, alkali metal salts such as lithium fluoride and cesium fluoride, alkaline earth metal salts such as magnesium fluoride, metal complexes of quinolinol derivatives such as lithium quinolinol, metal oxides such as aluminum oxide, or metals such as ytterbium (Yb), samarium (Sm), calcium (Ca), strontium (Sr), and cesium (Cs), can be used; however, in a preferred choice of electron transport layer and cathode, these can be omitted.

Furthermore, as materials for the electron injection layer or the electron transport layer, materials normally used for these layers in which an n-type metal dopant such as cesium is further doped can be used.

### [Cathode Electrode]

As materials for the cathode of the organic EL device of the invention, usable are electrode materials with a low work function such as aluminum, alloys with a lower work function such as magnesium silver alloys, magnesium calcium alloys, magnesium indium alloys and aluminum magnesium alloys, and conductive transparent materials such as ITO (indium tin oxide) and IZO (indium zinc oxide). Metals and alloys are formed in films having a thickness of approximately 10 to 200 nm to be semi-transparent cathode electrodes.

### [Capping Layer]

The organic EL device of the invention contains the aromatic compound represented by the general formula (a) or the general formula (b) in the capping layer. As described above, the capping layer preferably has a stack structure including a first capping layer containing the aromatic compound represented by the general formula (a) or the general formula (b) and a second capping layer with a higher refractive index than the first capping layer, and preferably, the capping layer has a two-layer structure of the first capping layer and the second capping layer. Preferably, the capping layer having a stack structure is arranged so that the first capping layer is adjacent to the cathode electrode. The capping layer and the first capping layer may contain only one of or two or more of the aromatic compounds represented by the general formula (a). The capping layer and the first capping layer may contain only one of or two or more of the aromatic compounds represented by the general formula (b). The capping layer and the first capping layer may contain only one of the aromatic compound represented by the general formula (a) and the aromatic compound represented by the general formula (b), or both.

For the second capping layer, a material with a higher refractive index than the aromatic compound used in the first capping layer is used. Arylamine compounds can be used as the material to be in the second capping layer (see, for example, PTLs 4 and 5).

Preferably, the refractive index of the material constituting the second capping layer is at least 0.15 greater than the refractive index of the adjacent first capping layer, more preferably at least 0.20 greater, even more preferably at least 0.30 greater.

Each of the first and second capping layers may be a single layer deposited with one of the above capping layer materials alone, or may be a mixed layer deposited with a mixture of the two or more materials. These materials can be formed into thin films by known methods such as vapor deposition, and in addition, spin-coating, or ink-jetting.

The total thickness of the first capping layer and the second capping layer is, for example, preferably 30 nm to 120 nm, more preferably 40 nm to 80 nm. The ratio of the thickness of the first capping layer to that of the second capping layer is preferably 1:99 to 99:1, more preferably 90:10 to 90:10, even more preferably 20:80 to 80:20. In that case, good light extraction efficiency is attained. The thicknesses of the first capping layer and the second capping layer can be appropriately changed according to the type of light-emitting material used in the light-emitting device, and the thickness of the other layers than the capping layer of the organic EL device.

The above description of the invention uses a top-emission type organic EL device as an example, but the organic EL device to which the invention is applied is not limited to this, that is, the organic EL device of the invention may also be an organic EL device with a bottom-emission structure, or may be a dual-emission structure that emits light from both the top and the bottom. For the description of the members and the layers that constitute the organic EL device with a bottom-emission structure or a dual-emission structure, reference can be made to the description of the organic EL device above. However, the electrode in the direction in which light is extracted externally from the light-emitting device need to be transparent or semi-transparent. In other words, in the bottom-emission structure, the electrode on the substrate side is preferably transparent or semi-transparent, and in the dual-emission structure, the electrodes on both sides are preferably transparent or semi-transparent. The capping layer can be arranged between the electrode on the substrate side and the substrate in the bottom emission structure, and in the dual emission structure, the capping layer can be arranged between the electrode on the substrate side and the substrate, and outside the electrode on the opposite side of the substrate, or only on one of them.

### <Electronic Device and Electronic Component>

The electronic device and the electronic component of the invention have a pair of electrodes and at least one organic layer, in which at least one of the at least one organic layer contains the aromatic compound represented by the general formula (a) or the general formula (b). For the description of the aromatic compound represented by the general formula (a) or the general formula (b), reference can be made to the description in the above section of "Aromatic Compound Represented by General Formula (a) or General Formula (b)".

Examples of the electronic device include displays and light-emitting devices equipped with an organic EL device, and additionally include electrophotographic photoreceptors, image sensors, photoelectric conversion devices, and solar cells. Examples of the display device include display components such as organic EL panel modules, and also TVs, cell phones, tablets and personal computers. Examples of the light-emitting device include lighting devices or vehicle lights.

### Examples

The following examples will specifically describe the embodiment of the invention, but the invention is not limited to the following examples, provided that the scope or spirit of the invention is not departed from. In these examples, the light emission characteristics of the organic EL device were measured at a current density of 10 mA/cm².

First, aromatic compounds represented by the general formula (a) or the general formula (b) were synthesized in the following Examples 1 to 8.

### [Example 1] Synthesis of Compound (1-7)

2,2'-Bis(4-aminophenyl)hexafluoropropane: 5.0 g, triethylamine: 6.4 g, and tetrahydrofuran: 100 mL were added to a reaction vessel, and stirred in an ice bath for 30 minutes. A mixture of 1-adamantane carbonyl chloride: 6.3 g and tetrahydrofuran: 20 mL was added dropwise to the reaction vessel over 30 minutes, and stirred for 3 hours. After raising the temperature to room temperature and stirring overnight, water: 100 mL and dichloromethane: 300 mL were added for extraction. The organic layer was concentrated, and the resulting solid was washed by sequential dispersion with 200 mL of methanol and 50 mL of dichloromethane to give 8.2 g (yield: 83.2%) of a white powder of (1-7).

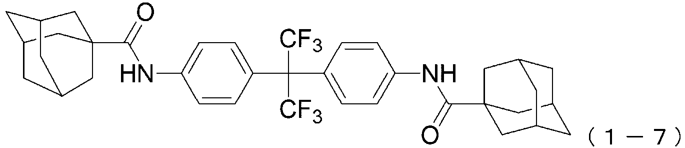

The structure of the resultant white powder was identified by NMR.

The following 40 hydrogen signals were detected in ¹H-NMR (CDCl₃). δ(ppm) = 7.55(4H), 7.36-7.31(6H), 2.11(6H), 1.96(12H), 1.76(12H).

### [Example 2] Synthesis of Compound (1-8)

2,2'-Bis(4-hydroxyphenyl)hexafluoropropane: 7.0 g, triethylamine: 4.9 g, and dichloromethane: 30 mL were added to a reaction vessel, and stirred in an ice bath for 30 minutes. A mixture of 1-adamantane carbonyl chloride: 8.7 g and dichloromethane: 20 mL was added dropwise to the reaction vessel over 30 minutes, and stirred for 3 hours. The temperature was raised to room temperature, and after stirring overnight, 50 mL of water was added, and the precipitated solid was collected by filtration. Methanol: 60 mL and water: 60 mL were added to wash the solid by dispersion, and cooled to room temperature, and the solid was collected by filtration. The resulting solid was washed with 130 mL of methanol to give 12.3 g (yield: 89.2%) of a white powder of (1-8).

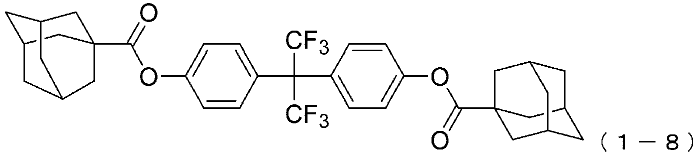

The structure of the resultant white powder was identified by NMR.

The following 38 hydrogen signals were detected in ¹H-NMR (CDCl₃). δ(ppm) = 7.39(4H), 7.08(4H), 2.09-2.06(18H), 1.77(12H).

### [Example 3] Synthesis of Compound (1-10)

1-Adamantamine hydrochloride: 5.1 g , triethylamine: 5.5 g, and tetrahydrofuran: 100 mL were added to a reaction vessel, and stirred in an ice bath for 30 minutes. A mixture of 4,4'-[2,2,2-trifluoro-1-(trifluoromethyl)ethylidene] bis[benzoyl chloride]: 5.5 g and tetrahydrofuran: 20 mL was added dropwise to the reaction vessel over 30 minutes, and stirred for 3 hours. After raising the temperature to room temperature and stirring overnight, water: 120 mL and dichloromethane: 300 mL were added for extraction. The organic layer was concentrated, and the resulting solid was washed by dispersion with 200 mL of methanol to give 6.6 g (yield: 78.2%) of a white powder of (1-10).

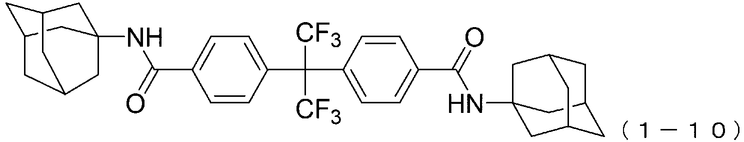

The structure of the resultant white powder was identified by NMR.

The following 40 hydrogen signals were detected in ¹H-NMR (CDCl₃). δ(ppm) = 7.71(4H), 7.41(4H), 5.79(2H), 2.12(18H), 1.72(12H).

### [Example 4] Synthesis of Compound (1-19)

1,1'-Bis(4-aminophenyl)cyclohexane: 4.0 g, triethylamine: 6.4 g, and tetrahydrofuran: 140 mL were added to a reaction vessel, and stirred in an ice bath for 30 minutes. A mixture of 1-adamantane carbonyl chloride: 6.3 g and tetrahydrofuran: 28 mL was added dropwise to the reaction vessel over 30 minutes, and stirred for 2 hours. The temperature was raised to room temperature, and after stirring for 3 hours, water was added, and the precipitated solid was collected by filtration. The resulting solid was washed by dispersion with methanol, and crystallized with THF to give 6.6 g (yield: 74.4%) of a white powder of (1-19).

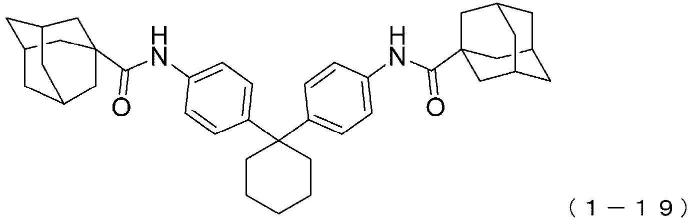

The structure of the resultant white powder was identified by NMR.

The following 50 hydrogen signals were detected in ¹H-NMR (CDCl₃). δ(ppm) = 7.43(4H), 7.22-7.18(6H), 2.20(4H), 2.09(6H), 1.94(12H), 1.75(12H), 1.53-1.47(6H).

### [Example 5] Synthesis of Compound (1-2)

2,2'-Bis(4-hydroxyphenyl)propane: 5.0 g, triethylamine: 4.9 g, and dichloromethane: 20 mL were added to a reaction vessel, and stirred in an ice bath for 30 minutes. A mixture of 1-adamantane carbonyl chloride: 9.2 g and dichloromethane: 15 mL was added dropwise to the reaction vessel over 30 minutes, and stirred for 2 hours. The temperature was raised to room temperature, and after stirring overnight, triethylamine: 0.6 g was added, stirred for 30 minutes in an ice bath, and a mixture of 1-adamantanecarbonyl chloride: 1.0 g and dichloromethane: 5 mL was dropwise added to the reaction vessel over 30 minutes, and stirred for 2 hours. The temperature was raised to room temperature, and after stirring for 2 hours, 60 mL of water was added, stirred for 1 hour, and the precipitated solid was collected by filtration. Methanol: 120 mL and water: 120 mL were added to wash the solid by dispersion, and the solid was collected by filtration. The crude product was crystallized with a mixed solvent of toluene/methanol to give 9.3 g (yield: 76.9%) of a white powder of (1-2).

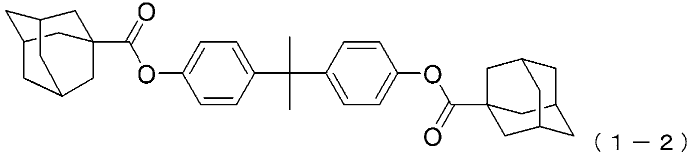

The structure of the resultant white powder was identified by NMR.

The following 44 hydrogen signals were detected in ¹H-NMR (CDCl₃). δ(ppm) = 7.20(4H), 6.93(4H), 2.08(6H), 2.04(12H), 1.76(12H), 1.65(6H).

### [Example 6] Synthesis of Compound (1-20)

1,1'-Bis(4-hydroxyphenyl)cyclohexane: 5.0 g, triethylamine: 4.1 g, and dichloromethane: 20 mL were added to a reaction vessel, and stirred in an ice bath for 30 minutes. A mixture of 1-adamantane carbonyl chloride: 7.8 g and dichloromethane: 10 mL was added dropwise to the reaction vessel over 30 minutes, and stirred for 2 hours. After raising the temperature to room temperature and stirring overnight, water: 100 mL and dichloromethane: 200 mL were added for extraction. The organic layer was concentrated, and the resulting solid was crystallized with a mixed solvent of dichloromethane/methanol to give 9.1 g (yield: 82.4%) of a white powder of (1-20).

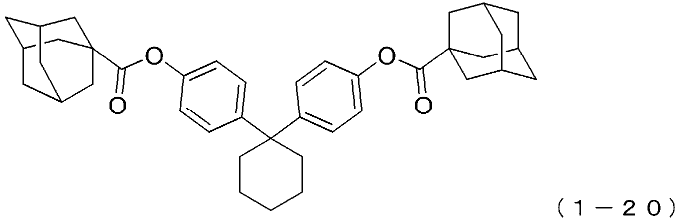

The structure of the resultant white powder was identified by NMR.

The following 48 hydrogen signals were detected in ¹H-NMR (CDCl₃). δ(ppm) = 7.23(4H), 6.94(4H), 2.24(4H), 2.07(6H), 2.03(12H), 1.76(12H), 1.55-1.49(6H).

### [Example 7] Synthesis of Compound (1-193)

1-Adamantane methylamine: 4.4 g, triethylamine: 5.4 g, and tetrahydrofuran: 120 mL were added to a reaction vessel, and stirred in an ice bath for 30 minutes. A mixture of 4,4'-[2,2,2-trifluoro-1-(trifluoromethyl)ethylidene] bis[benzoyl chloride]: 5.8 g and tetrahydrofuran: 20 mL mixture was added dropwise to the reaction vessel over 30 minutes, and stirred for 3 hours. The temperature was raised to room temperature, and after stirring overnight, 150 mL of water was added for washing by dispersion, and the solid was collected by filtration. The resulting crude product was crystallized with a mixed solvent of tetrahydrofuran/methanol to give 5.5 g (yield: 62.6%) of a white powder of (1-193).

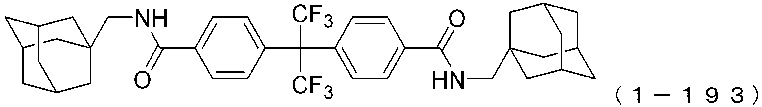

The structure of the resultant white powder was identified by NMR.

The following 44 hydrogen signals were detected in ¹H-NMR (CDCl₃). δ(ppm) = 7.77(4H), 7.45(4H), 6.16(2H), 3.17(4H), 2.00(6H), 1.75-1.56(24H).

### [Example 8] Synthesis of Compound (1-194)

3,5-Dimethyl-1-adamantamine hydrochloride: 5.8 g , triethylamine: 5.4 g, and tetrahydrofuran: 120 mL were added to a reaction vessel, and stirred in an ice bath for 30 minutes. A mixture of 4,4'-[2,2,2-trifluoro-1-(trifluoromethyl)ethylidene] bis[benzoyl chloride]: 5.9 g and tetrahydrofuran: 20 mL mixture was added dropwise to the reaction vessel over 30 minutes, and stirred for 3 hours. The temperature was raised to room temperature, and after stirring overnight, 150 mL of water was added for washing by dispersion, and the solid was collected by filtration. The resulting crude product was crystallized with a mixed solvent of tetrahydrofuran/methanol to give 4.0 g (yield: 44.2%) of a white powder of (1-194).

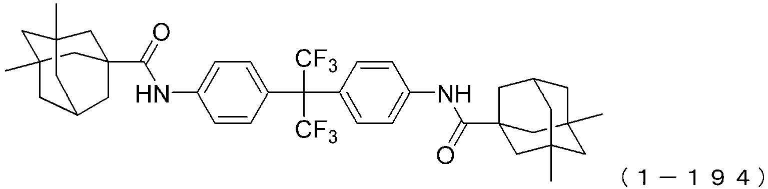

The structure of the resultant white powder was identified by NMR.

The following 48 hydrogen signals were detected in ¹H-NMR (CDCl₃). δ(ppm) = 7.70(4H), 7.40(4H), 5.81(2H), 2.20(2H), 1.95(4H), 1.76(8H), 1.45-1.19(12H), 0.88(12H).

### [Example 9] Measurement of Glass Transition Point and Melting Point

The glass transition point and the melting point of the aromatic compounds obtained in Examples 1 to 8 were measured with a high-sensitivity differential scanning calorimeter (DSC 3100SA, manufactured by Bruker AXS). The measurement results are summarized in Table 1.

**[Table 1]**

| Compound | Glass Transition Point [°C] | Melting Point [°C] |
|---|---|---|
| Compound (1-7) | 165 | 296 |
| Compound (1-8) | - | 294 |
| Compound (1-10) | 155 | 263 |
| Compound (1-19) | 162 | 328 |
| Compound (1-2) | - | 291 |
| Compound (1-20) | 95 | 251 |
| Compound (1-193) | 149 | 268 |
| Compound (1-194) | 133 | 254 |

### [Example 10] Measurement of Refractive Index

Using the aromatic compounds obtained in Examples 1 to 8, a vapor-deposited film with a thickness of 80 nm was prepared on a silicon substrate, and the refractive index n thereof was measured at room temperature using a spectrometer (F10-RT-UV, manufactured by Filmetrics, Inc.). The refractive index n was also measured in the same way for Compound (CPL-1) used as the high refractive index capping material in the following organic EL device fabrication example and for Alq₃ used as a comparative compound. The measurement results are summarized in Table 2.

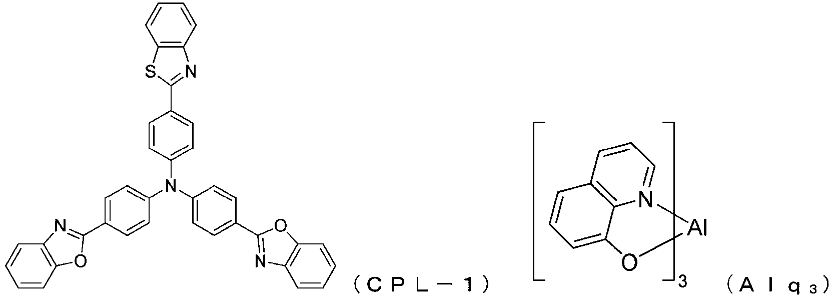

**[Table 2]**

| | Refractive Index n (λ: 400nm) | Refractive Index n (λ: 700nm) |
|---|---|---|
| Compound (1-7) | 1.64 | 1.56 |
| Compound (1-8) | 1.07 | 1.03 |
| Compound (1-10) | 1.65 | 1.55 |
| Compound (1-19) | 1.68 | 1.59 |
| Compound (1-2) | 1.11 | 1.11 |
| Compound (1-20) | 1.17 | 1.21 |
| Compound (1-193) | 1.58 | 1.49 |
| Compound (1-194) | 1.55 | 1.46 |
| Alq3 | 1.86 | 1.74 |
| CPL-1 | 2.39 | 1.91 |

As shown in Table 2, the refractive indices of the aromatic compounds of the invention in the wavelength range from 400 nm to 700 nm are not more than 1.70, which are smaller than the refractive indices of Alq₃ and Compound (CPL-1).

### [Example 11] Fabrication of Organic EL Device using Compound (1-7)

As shown in Figure 1, an organic EL device was fabricated by depositing a hole injection layer 3, a hole transport layer 4, a light-emitting layer 5, an electron transport layer 6, an electron injection layer 7, a cathode 8, a first capping layer 9 and a second capping layer10 in this order on a glass substrate 1 with a reflective ITO electrode preformed as a transparent anode 2.

Specifically, a glass substrate 1 having ITO with a thickness of 50 nm, a silver alloy reflective film with a thickness of 100 nm, and ITO with a thickness of 5 nm in this order was ultrasonically cleaned in isopropyl alcohol for 20 minutes and then dried on a hot plate heated at 250°C for 10 minutes. Then, after UV ozone treatment for 2 minutes, this glass substrate with ITO was mounted in a vacuum evaporator and the pressure was reduced to 0.001 Pa or less. Next, an electron acceptor of the following structural formula (Acceptor-1) and a compound of the following structural formula (3-1) were deposited to form a hole injection layer 3 having a thickness of 10 nm and covering the transparent anode 2, by binary deposition at a deposition rate of (Acceptor-1):Compound (3-1) = 3:97. A compound of the following structural formula (3-1) was formed as the hole transport layer 4 on top of the hole injection layer 3 to achieve a film thickness of 140 nm. On top of the hole transport layer 4, a compound of the following structural formula (EMD-1) and a compound of the following structural formula (EMH-1) were deposited to form a light-emitting layer 5 with a thickness of 20 nm, by binary deposition at a deposition rate ratio of (EMD-1): (EMH-1) = 5:95. On top of the light-emitting layer 5, a compound of the following structural formula (4-1) and a compound of the following structural formula (ETM-1) were deposited to form an electron transport layer 6 with a thickness of 30 nm, by binary deposition at a deposition rate ratio of (4-1): (ETM-1) = 50:50. On top of the electron transport layer 6, lithium fluoride was formed as an electron injection layer 7 with a thickness of 1 nm.

On the electron injection layer 7, a magnesium silver alloy was formed as a cathode 8 with a thickness of 12 nm. Compound (1-7) of Example 1 was formed as the first capping layer 9 on top of the cathode 8 to a thickness of 30 nm, and finally compound (CPL-1) was formed as the second capping layer 10 to a thickness of 30 nm. The light emission characteristics of the fabricated organic EL devices were measured by applying a DC voltage to the devices in air at room temperature. The measurement results are summarized in Table 3.

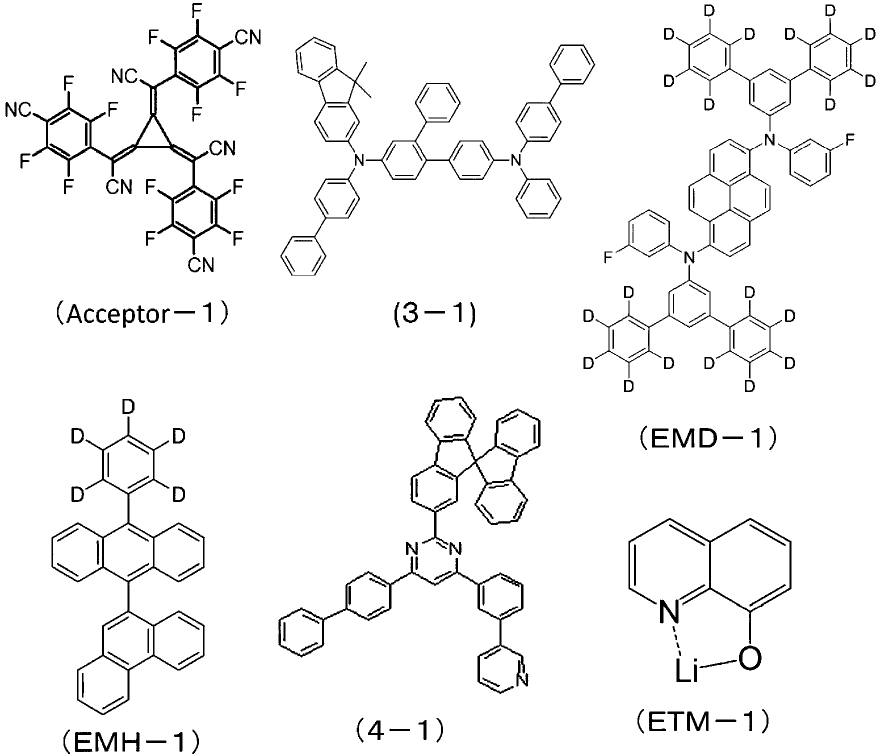

### [Example 12] Fabrication of Organic EL Device using Compound (1-8)

An organic EL device was fabricated under the same conditions as in Example 11, except that compound (1-8) was used instead of compound (1-7) as the material for the first capping layer 9 and the first capping layer 9 was formed to have a thickness of 30 nm. The light emission characteristics of the fabricated organic EL devices were measured by applying a DC voltage to the devices in air at room temperature. The measurement results are summarized in Table 3.

### [Example 13] Fabrication of Organic EL Device using Compound (1-10)

An organic EL device was fabricated under the same conditions as in Example 11, except that compound (1-10) was used instead of compound (1-7) as the material for the first capping layer 9 and the first capping layer 9 was formed to have a thickness of 30 nm. The light emission characteristics of the fabricated organic EL devices were measured by applying a DC voltage to the devices in air at room temperature. The measurement results are summarized in Table 3.

### [Example 14] Fabrication of Organic EL Device using Compound (1-19)

An organic EL device was fabricated under the same conditions as in Example 11, except that compound (1-19) was used instead of compound (1-7) as the material for the first capping layer 9 and the first capping layer 9 was formed to have a thickness of 30 nm. The light emission characteristics of the fabricated organic EL devices were measured by applying a DC voltage to the devices in air at room temperature. The measurement results are summarized in Table 3.

### [Example 15] Fabrication of Organic EL Device using Compound (1-2)

An organic EL device was fabricated under the same conditions as in Example 11, except that compound (1-2) was used instead of compound (1-7) as the material for the first capping layer 9 and the first capping layer 9 was formed to have a thickness was 30 nm. The light emission characteristics of the fabricated organic EL devices were measured by applying a DC voltage to the devices in air at room temperature. The measurement results are summarized in Table 3.

### [Example 16] Fabrication of Organic EL Device using Compound (1-20)

An organic EL device was fabricated under the same conditions as in Example 11, except that compound (1-20) was used instead of compound (1-7) as the material for the first capping layer 9 and the first capping layer 9 was formed to have a thickness was 30 nm. The light emission characteristics of the fabricated organic EL devices were measured by applying a DC voltage to the devices in air at room temperature. The measurement results are summarized in Table 3.

### [Example 17] Fabrication of Organic EL Device using Compound (1-193)

An organic EL device was fabricated under the same conditions as in Example 11, except that compound (1-193) was used instead of compound (1-7) as the material for the first capping layer 9 and the first capping layer 9 was formed to have a thickness was 30 nm. The light emission characteristics of the fabricated organic EL devices were measured by applying a DC voltage to the devices in air at room temperature. The measurement results are summarized in Table 3.

### [Example 18]

An organic EL device was fabricated under the same conditions as in Example 11, except that compound (1-194) was used instead of compound (1-7) as the material for the first capping layer 9 and the first capping layer 9 was formed to have a thickness was 30 nm. The light emission characteristics of the fabricated organic EL devices were measured by applying a DC voltage to the devices in air at room temperature. The measurement results are summarized in Table 3.

### [Comparative Example 1] Fabrication of Organic EL Device using ALq₃

An organic EL device was fabricated under the same conditions as in Example 11, except that ALq₃ was used instead of compound (1-7) as the material for the first capping layer 9 and the first capping layer was formed to have a thickness of 30 nm. The light emission characteristics of the fabricated organic EL devices were measured by applying a DC voltage to the devices in air at room temperature. The measurement results are summarized in Table 3.

### [Comparative Example 2] Fabrication of Organic EL Device with single capping layer

An organic EL device was fabricated under the same conditions as in Example 11, except that Compound (CPL-1) was used to form a single capping layer having a thickness of 60 nm, instead of the capping layer formed of the first capping layer 9 and the second capping layer 10. The light emission characteristics of the fabricated organic EL devices were measured by applying a DC voltage to the devices in air at room temperature. The measurement results are summarized in Table 3.

The results of device lifetime measured with the organic EL devices fabricated in Example 11 to Example 18, and Comparative Examples 1 and 2 are summarized in Table 3. The device lifetime was expressed as the time required for the luminance to decay to 95% of the initial luminance when the device was driven at a constant current of 10 mA/cm².

**[Table 3]**

| | First Capping Layer | Second Capping Layer | Voltage [V] (@10mA /cm²) | Luminance [cd/m²] (@10mA/cm²) | Light Emission Efficiency [cd/A] (@10mA/cm²) | Power Efficiency [lm/W] (@10mA/cm²) | Device Lifetime 95% Decay |
|---|---|---|---|---|---|---|---|
| Ex. 11 | Cmpd (1-7) | CPL-1 | 3.64 | 843 | 8.43 | 7.28 | 171 hrs. |
| Ex. 12 | Cmpd (1-8) | CPL-1 | 3.65 | 849 | 8.49 | 7.31 | 173 hrs. |
| Ex. 13 | Cmpd (1-10) | CPL-1 | 3.64 | 842 | 8.42 | 7.27 | 168 hrs. |
| Ex. 14 | Cmpd (1-19) | CPL-1 | 3.63 | 843 | 8.43 | 7.29 | 170 hrs. |
| Ex. 15 | Cmpd (1-2) | CPL-1 | 3.63 | 828 | 8.28 | 7.16 | 175 hrs. |
| Ex. 16 | Cmpd (1-20) | CPL-1 | 3.64 | 827 | 8.27 | 7.13 | 171 hrs. |
| Ex. 17 | Cmpd (1-193) | CPL-1 | 3.65 | 831 | 8.31 | 7.17 | 178 hrs. |
| Ex. 18 | Cmpd (1-194) | CPL-1 | 3.63 | 831 | 8.31 | 7.21 | 170 hrs. |
| Comp Ex. 1 | Alq₃ | CPL-1 | 3.64 | 817 | 8.17 | 7.05 | 163 hrs. |
| Comp Ex. 2 | - | CPL-1 | 3.65 | 794 | 7.93 | 6.84 | 159 hrs. |

| | | | | | | | |
|---|---|---|---|---|---|---|---|
| Ex.: Example, Comp Ex.: Comparative Example, Cmpd: Compound, hrs.: hours | | | | | | | |

As shown in Table 3, the drive voltage at a current density of 10 mA/cm² was almost the same for the devices of Comparative Examples 1 and 2 and those of Examples 11 to 18. In contrast, the devices in Examples 11 to 18 were all improved in terms of luminance, light emission efficiency, power efficiency, and lifetime compared to the devices in Comparative Examples 1 and 2. This indicates that the light extraction efficiency can be significantly improved by providing a stack structure of a first capping layer containing the aromatic compound of the invention and a second capping layer with a high refractive index as a capping layer.

### Industrial Applicability

The organic EL device with a capping layer that contains the aromatic compound of the invention, especially the organic EL device with a capping layer having a stack structure of a first capping layer containing the aromatic compound of the invention and a second capping layer with a high refractive index can attain high efficiency and therefore can be effectively used in various devices that use light-emitting devices. Accordingly, the industrial applicability of the invention is great.

### Reference Signs List

1 Glass Substrate
2 Transparent Anode
3 Hole Injection Layer
4 Hole Transport Layer
5 Light-Emitting Layer
6 Electron Transport Layer
7 Electron Injection Layer
8 Cathode
9 First Capping Layer
10 Second Capping Layer

## Claims

1. An aromatic compound represented by the following general formula (a) or the following general formula (b): 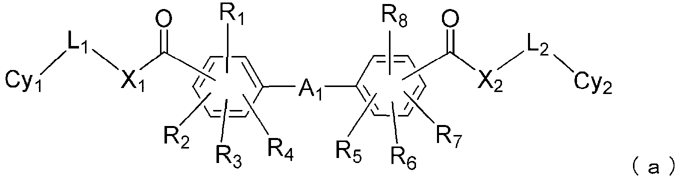 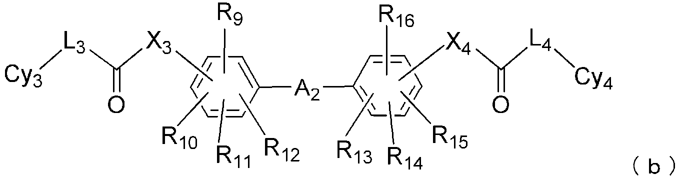
wherein A₁ and A₂ each independently represent a substituted or unsubstituted alkanediyl group, a substituted or unsubstituted cycloalkanediyl group, or a substituted or unsubstituted fluorenediyl group,
L₁ to L₄ each independently represent a single bond, -O-, -NH-, or a substituted or unsubstituted alkanediyl group,
R₁ to R₁₆ each independently represent a hydrogen atom, a halogen atom, a substituted or unsubstituted alkyl group, a substituted or unsubstituted cycloalkyl group, a substituted or unsubstituted monovalent aromatic hydrocarbon group, or a substituted or unsubstituted monovalent aromatic heterocyclic group,
Cy₁ to Cy₄ each independently represent a substituted or unsubstituted alkyl group, a substituted or unsubstituted cycloalkyl group, or a substituted or unsubstituted monovalent aromatic hydrocarbon group, and
X₁ to X₄ each independently represent -O- or -NH-.

2. The aromatic compound according to claim 1, wherein R₁ to R₈ in the general formula (a) and R₉ to R₁₆ in the general formula (b) are each independently a hydrogen atom, a halogen atom, a substituted or unsubstituted alkyl group, or a substituted or unsubstituted cycloalkyl group.

3. The aromatic compound according to claim 2, wherein A₁ in the general formula (a) and A₂ in the general formula (b) are each independently a divalent group represented by the following formula (c), formula (d), formula (e), formula (f), or formula (g): 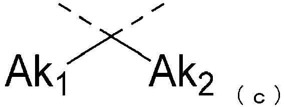 wherein Ak₁ and Ak₂ each independently represent a hydrogen atom, an unsubstituted alkyl group, or an unsubstituted haloalkyl group, and the dashed portions represent bonding sites, 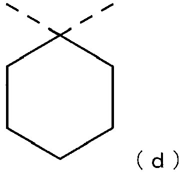 wherein the dashed portions represent bonding sites, 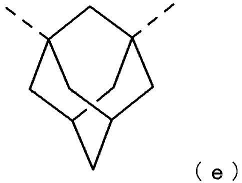 wherein the dashed portions represent bonding sites, 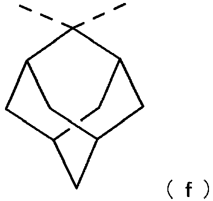 wherein the dashed portions represent bonding sites, 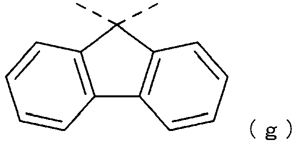 wherein the dashed portions represent bonding sites.

4. The aromatic compound according to claim 3, wherein L₁ and L₂ in the general formula (a) and L₃ and L₄ in the general formula (b) each independently represent a single bond, -O-, -NH-, a substituted or unsubstituted methylene group, a substituted or unsubstituted ethanediyl group, or a substituted or unsubstituted propanediyl group.

5. The aromatic compound according to claim 4, wherein Cy₁ and Cy₂ in the general formula (a) and Cy₃ and Cy₄ in the general formula (b) each independently represent a substituted or unsubstituted methyl group, a substituted or unsubstituted cyclohexyl group, a substituted or unsubstituted norbornyl group, a substituted or unsubstituted adamantyl group, or a substituted or unsubstituted phenyl group.

6. An organic EL device having at least an anode electrode, a hole transport layer, a light-emitting layer, an electron transport layer, a cathode electrode, and a capping layer in this order, in which the capping layer contains the aromatic compound of any of claims 1 to 5.

7. The organic EL device according to claim 6, wherein the aromatic compound exhibits a refractive index of 1.70 or less at room temperature for light with a wavelength of 400 nm or more to 700 nm or less when vacuum-deposited on a silicon substrate at a film thickness of 80 nm to form a vapor-deposited film.

8. The organic EL device according to claim 6, wherein the capping layer is a stacked or mixed layer including two or more compounds, and at least one compound in the capping layer is the aromatic compound.

9. An electronic component or an electronic device having a pair of electrodes and at least one organic layer, in which the organic layer contains an aromatic compound of any of claims 1 to 5.
